# EUROPÄISCHE PATENTANMELDUNG

(11) **EP 4 807 369 A1**
(43) Veröffentlichungstag der Anmeldung: **16.09.2026**
(21) Anmeldenummer: 26163126.1
(22) Anmeldetag: 09.03.2026
(51) Int. Cl.: G01N 33/58, G01N 33/543

(54) **VERFAHREN ZUM NACHWEIS VON ANALYTEN UND DESSEN VERWENDUNG**

(30) Priorität: 10.03.2025 DE 102025108930
(71) Anmelder: Universität Leipzig, Körperschaft des öffentlichen Rechts, 04109 Leipzig (DE)
(72) Erfinder: Pompe, Tilo, 04109 Leipzig (DE); Riedl, Veronika, 04109 Leipzig (DE); Gehring, Rosa, 04109 Leipzig (DE)
(74) Vertreter: Kailuweit & Uhlemann Patentanwälte Partnerschaft mbB

(57) **Zusammenfassung**

Die Erfindung betrifft ein Verfahren zum Nachweis eines Analyten mittels eines immobilisierten Analytbindungspartners und deformierbarer Partikel und dessen Verwendung zum Nachweis von Analyten wie Pestiziden, hormonell aktiven Verbindungen, Metallpartikeln, Metallionen, Viren, Bakterien, Biomarkern oder Antibiotika in Lebensmittel-, Futtermittel-, Waschmittel-, Spülmittel-, Körperpflegemittel-, Kosmetik-, Pharmaka-, Prozesswasser-, Boden-, Gewässer-, Trinkwasserund/oder Abwasserproben und/oder Körperflüssigkeiten.

## Beschreibung

Die Erfindung betrifft ein Verfahren zum Nachweis eines Analyten mittels eines immobilisierten Analytbindungspartners und deformierbarer Partikel und dessen Verwendung zum Nachweis von Analyten wie Pestiziden, hormonell aktiven Verbindungen, Metallpartikeln, Metallionen, Viren, Bakterien, Biomarkern oder Antibiotika in Lebensmittel-, Futtermittel-, Waschmittel-, Spülmittel-, Körperpflegemittel-, Kosmetik-, Pharmaka-, Prozesswasser-, Boden-, Gewässer-, Trinkwasser- und/oder Abwasserproben und/oder Körperflüssigkeiten.

Der Bedarf an robuster und kostengünstiger Analytik steigt seit Jahren, u.a. in den Bereichen der medizinischen Diagnostik, Biotechnologie, Agrarwissenschaften und Umweltdiagnostik.

Ein zunehmender Grund für den Bedarf besteht neben der Zunahme des Umweltbewusstseins in der öffentlichen Diskussion des Vorkommens von Schadstoffen, beispielsweise Pestiziden, Antibiotika oder hormonell aktiven Verbindungen, in Lebensmittel- bzw. Trinkwasser- und Umweltproben. Eine wesentliche Voraussetzung sowohl für die Verfolgung der Eintragswege als auch den Schutz der Bevölkerung vor zu hoher Belastung mit Schadstoffen ist eine einfache und umfassend anwendbare Analytik.

Neben der höchstmöglichen Genauigkeit bei der Analytik spielt auch der wirtschaftliche Faktor eine entscheidende Rolle. Daher werden neue Methoden und Verfahren zur Detektion und Charakterisierung von Analyten benötigt, die kostengünstig sind und eine schnelle Analyse ermöglichen.

Momentan erfordert der Nachweis von vielen anthropogenen Spurenstoffen, wie Pestiziden, hormonell aktiven Verbindungen oder Antibiotika, eine Probennahme, in vielen Fällen den Transport in ein geeignetes Labor zum Nachweis, eine aufwendige Probenvorbereitung und den eigentlichen Nachweis, z.B. mittels ELISA (*Enzyme-linked Immunosorbent Assay*), LC (*Liquid chromatography*)- oder GC (*Gas chromatography*)*-*Verfahren mit anschließender Massenspektrometrie, Fluorimetrie oder Photometrie, über zellbasierte Rezeptortests oder durch mikrobiologische Nachweisverfahren. Diese Verfahren sind sowohl zeit- als auch materialaufwendig.

WO 2008 118 899 A1 offenbart ein Verfahren zum Nachweis von Glyphosat mittels LC/GC gekoppeltem MS (Massenspektrometrie). Alternativ sind auch Nachweismethoden mittels Biolumineszenz (WO 2010 104 861 A1) und ELISA (WO 2000 014 538 A1) bekannt.

Insbesondere bei kleinen Analyten wird häufig ein indirekter Nachweis gewählt. Der üblichste indirekte Test ist ein ELISA, ein antikörperbasiertes Nachweisverfahren (Assay), wobei eine Quantifizierung mittels einer enzymatischen Farbreaktion erfolgt. Nachteilig ist ein ELISA von der Verfügbarkeit geeigneter Antikörper abhängig.

Ein robustes Verfahren für eine Vor-Ort-Analytik wird durch Bittner et al. beschrieben, wobei die Einbettung von gentechnisch modifizierten Hefezellen von *Saccharomyces cerevisiae* in eine Polymermatrix, insbesondere Gelatine und Agar, zur Herstellung eines Hefe-Assays zum Nachweis von Östrogenen und Androgenen genutzt wird, welcher außerhalb des Labors ohne Vorbereitungszeiten einsetzbar ist (Bittner et al. 2015). Bittner et al. beschreiben die Durchführung des Hefe-Assays zum Nachweis von Testosteron und 17β-Estradiol in 3 h durch Verwendung der immobilisierten Hefezellen.

EP 2 752 664 A1 bzw. WO 2014/106 665 A1 offenbart ein Verfahren zur Detektion von Analyten mittels der Immobilisierung eines Analyten an Hydrogelpartikel, welche nachfolgend mit einem auf einer Oberfläche immobilisierten Liganden interagieren. Abhängig vom Grad der Interaktion erfolgt eine Deformierung der Hydrogelpartikel durch Anlagerung an die Oberfläche, sodass die Deformierung mittels Reflexionsinterferenzkontrastmikroskopie gemessen werden kann. Dadurch kann eine Detektion bzw. Charakterisierung des Analyten erfolgen.

Rettke et al. bzw. DE 10 2018 130 134 A1 beschreibt ein kompetitives Nachweisverfahren für Glyphosat, mittels einer Oberfläche, funktionalisiert mit einer 5-Enolpyruvylshikimat-3-phosphat-Synthase, und mit einem deformierbaren Partikel, funktionalisiert mit Phosphoenolpyruvat, Phosametin oder Glyphosat als Kompetitor, wobei zur Funktionalisierung der Oberfläche bevorzugt ein Fusionsprotein mit einer Hydrophobin-Domäne verwendet wird (Rettke et al. 2020). Die Detektion erfolgt durch Quantifizierung der Deformation des Partikels an der Oberfläche. Mittels spezieller mikroskopischer Verfahren, bspw. der Reflexionsinterferenzkontrastmikroskopie (RICM), Quarzkristallmikrowaage (QCM), Oberflächenplasmonenresonanzspektroskopie (SPR), Rasterkraftspektroskopie (AFM) oder Impedanzspektroskopie, wird die Kontaktfläche zwischen Partikel und Oberfläche ermittelt. Absatz [0031] beschreibt insbesondere die Abhängigkeit der Kontaktfläche zwischen Partikel und Oberfläche von der Analytkonzentration in der Probe.

DE 10 2004 062 573 A1 offenbart Funktionselemente, die auf einem Träger angeordnete, biofunktionalisierte Nanopartikel enthaltende Mikrostrukturen umfassen, Verfahren zur Herstellung dieser Funktionselemente sowie die Verwendung derselben. Die Mikrostrukturen werden aus mehreren dreidimensional übereinander angeordneten Schichten von Nanopartikeln gebildet und die biofunktionalisierten Nanopartikel weisen molekülspezifische Erkennungsstellen auf, an welche bevorzugt ein oder mehrere biologisch aktive Moleküle gebunden sind. Die biologisch aktiven Moleküle werden als Proteine, insbesondere Antikörper, Antigene, Enzyme, Cytokine, Rezeptoren oder Strukturproteine; Proteinkomplexe, Nukleinsäuren, PNA-Moleküle, Fragmente davon und/oder eine Kombination davon beschrieben. Bevorzugt ist zwischen der Trägeroberfläche und der Mikrostruktur eine Schicht eines Verbindungsmittels angeordnet und das Verbindungsmittel ist bevorzugt ein Polymer, insbesondere ein Hydrogel. Die Mikrostruktur (umfassend bevorzugt ein Hydrogel) hat dabei die Funktion der Vergrößerung der Oberfläche und damit die zur Verfügung stehende Reaktionsfläche Stabilisierung der Erkennungsstellen auf der Oberfläche. Als Messmethoden werden Matrixunterstützte Laser-Desorptions/lonisations-Flugzeit-Massenspektrometrie (MALDI-TOF-MS), Wellenleiterspektroskopie, Fluoreszenz, Impedanz-spektroskopie, radiometrische und elektrische Verfahren genannt.

WO 2022/039 594 A1 beschreibt eine Biosensorvorrichtung zum Erfassen eines Analyten über einen Zeitraum von etwa 10 min unter Verwendung von Brownschen Partikelbewegungen (Diffusion), wobei die Biosensorvorrichtung eine Oberfläche und ein Partikel aufweist, wobei das Partikel und/oder die Oberfläche funktionalisiert sind und das Partikel nicht an die Oberfläche konjugiert ist. In einem ersten Zustand ist das Partikel mit der Oberfläche verbunden, und in einem zweiten Zustand ist das Partikel nicht mit der Oberfläche verbunden, wobei der Abstand zwischen dem Partikel und dem der Oberfläche im zweiten Zustand vorzugsweise im Bereich von 5 nm bis 10 µm liegt. Das Umschalten zwischen dem ersten und zweiten Zustand ist von der Anwesenheit, Abwesenheit und/oder Konzentration des Analyten abhängig. Dadurch kann die Anwesenheit des Analyten durch die Änderung eines räumlichen Koordinatenparameters des Partikels relativ zur Oberfläche gemessen werden, insbesondere durch Bestimmung der Partikeldiffusion mittels Strahlung, Wellen, elektromagnetischen Prinzipien, Akustik, Streuung, Fluoreszenz, Absorption, Interferenz, plasmonischer Erfassung, spektroskopischer Erfassung, Bildgebung usw. Dabei muss insbesondere die Bewegung der Partikel (typisch mindestens 100 Partikel) sehr genau über den gesamten Zeitraum gemessen werden, um daraus das Bewegungsprofil und den zugehörigen Einfluss des Analyten zu bestimmen.

Nachteilig sind die bekannten Verfahren gerätetechnisch äußerst aufwendig.

Aufgabe ist es daher, ein Verfahren zum Nachweis eines Analyten anzugeben, welches eine vereinfachte Detektion aufweist.

Erfindungsgemäß wird die Aufgabe durch das Verfahren und das Kit gemäß dem unabhängigen Anspruch gelöst. Vorteilhafte Ausgestaltungen der Erfindung sind in den abhängigen Ansprüchen angegeben.

Ein erster Aspekt der Erfindung betrifft ein Verfahren zum Nachweis eines Analyten umfassend die Schritte:
a) Bereitstellen mindestens einer Sensoroberfläche mit einem immobilisierten Analytbindungspartner,
   wobei der Analytbindungspartner fähig ist, mit einem Analyten zu interagieren,
b) Bereitstellen einer Probe enthaltend den Analyten, wobei die Probe als Lösung oder Dispersion vorliegt,
c) Kontaktieren der Sensoroberfläche mit der Probe,
   wobei der Analytbindungspartner mit dem Analyten interagiert,
d) Kontaktieren der Sensoroberfläche mit einem deformierbaren Partikel, wobei das deformierbare Partikel mit einem Interaktionspartner modifiziert ist, sodass in Abhängigkeit der Konzentration des Analyten eine Interaktion zwischen Partikel und Sensoroberfläche erfolgt,
e) Neigung der Sensoroberfläche in einem Winkel im Bereich von 30° bis 150°, ausgehend von einer horizontalen Lage der Sensoroberfläche, und
f) Detektion des Analyten durch optische Bestimmung der Bewegung des deformierbaren Partikels entlang der Sensoroberfläche.

Vorteilhafterweise ermöglicht das Verfahren eine einfache optische Bestimmung der Wechselwirkung, d. h. der Bindung, zwischen dem Partikel (Interaktionspartner) und der Sensoroberfläche (Analytbindungspartner) in Abhängigkeit der Konzentration des Analyten. Hierzu bedient sich die Erfindung eines zweistufigen Verfahrens. Während in den Schritten a) bis d) Analytbindungspartner, Analyt und der an das Partikel gebundene Interaktionspartner mit der Sensoroberfläche in einer vorzugsweise horizontalen Position interagieren, erfolgt die eigentliche Detektion erst nach der Neigung der Sensoroberfläche, in Schritt e) und f). Die Neigung kann in Abhängigkeit der Wechselwirkung zwischen Partikel und Sensoroberfläche zur Bewegung der in Schritt d) an die Sensoroberfläche gebundenen Partikel entlang der Sensoroberfläche führen. Die mögliche Sedimentationsbewegung wird durch die Gravitationskraft auf das Partikel und deren Differenz zum Auftrieb des Partikels ausgelöst. Die Konzentration des Analyten in der Probe bestimmt hierbei, ob sich das Partikel parallel zur geneigten Sensorsensoroberfläche bewegt. Ist die Sedimentationskraft größer als die Adhäsionskraft (des Partikels an der Sensoroberfläche), bewegt sich das Partikel in vertikaler Richtung entlang der Sensoroberfläche. Diese Bewegung kann optisch, bspw. mittels der Abfolge von zwei mikroskopischen Bildern, detektiert werden. Aufwändige Detektionsschritte zur Quantifizierung der Kontaktflächen des Partikels entfallen, wodurch der Auswerteaufwand wesentlich minimiert wird.

Vorteilhaft kann die Bewegung der Partikel entlang der Sensoroberfläche über eine Funktionalisierung der Partikel und/oder der Sensoroberfläche eingestellt werden. Weiterhin wirken sich Eigenschaften des Partikels (Durchmesser und Elastizität) auf die Adhäsionskraft aus, sodass mittels des Verfahrens vorteilhafterweise unterschiedliche Grenzwerte der Analyt-Konzentration bestimmt werden können.

Darüber hinaus eignet sich das Verfahren insbesondere für eine miniaturisierte, kostengünstige und mobile Auslesung, da auf den Einsatz komplizierter Messtechnik verzichtet wird. Die Größe der Partikel ermöglicht auch eine einfache visuelle Detektion, sodass zusätzliche Hardware für die Detektion bzw. Auswertung nicht nötig ist.

Weiterhin ermöglicht das Verfahren einen sehr schnellen Nachweis. Bereits nach einem Zeitraum von 1 bis 5 Minuten sind die Partikel in Schritt d) an der Sensoroberfläche gebunden und die Schritte e) und f) können ausgeführt werden. In Abhängigkeit der ausgewählten Detektionsmethodik kann eine Aussage über die Bewegung der Partikel schon eine Minute nach der Neigung der Sensoroberfläche getroffen werden.

Vorteilhaft ermöglicht das erfindungsgemäße Verfahren mithin eine einfache, schnelle und kostengünstige Vor-Ort-Analytik für Analyten wie Pestizide (Glyphosat), hormonell aktive Verbindungen oder Antibiotika, wodurch die Messung und Kontrolle von Schadstoffen (Umweltmonitoring), verbessert wird.

In Ausführungsformen erfolgt das erfindungsgemäße Verfahren mit einer Reihenfolge der Schritte a) und b), c), d), e) und f), wobei die Schritte a) und b) gleichzeitig oder in der Reihenfolge a) und danach b) oder b) und danach a) erfolgen. In Ausführungsformen erfolgt eine gleichzeitige Kontaktierung der Sensoroberfläche mit dem immobilisierten Analytbindungspartner mit dem Analyten (Schritt c) und dem an das Partikel gebundenen Interaktionspartner (Schritt d). Zweckmäßig erfolgt Schritt e) nach den Schritten c) und d) und Schritt f) nach Schritt e).

Erfindungsgemäß erfolgt ein Nachweis von Analyten, welche fähig sind, mindestens mit einem Analytbindungspartner zu interagieren. Unter dem Begriff "interagieren" wird eine reversible Wechselwirkung, insbesondere aufgrund von koordinativen Bindungen, Ionenbindungen, Wasserstoffbrückenbindungen, Van-der-Waals-Kräften und hydrophoben Effekten verstanden.

Zweckmäßig interagieren die Partikel mit der Sensoroberfläche (siehe Schritt d)) über eine Interaktion des Interaktionspartners mit dem Analytbindungspartner (kompetitives Verfahren) oder über eine Interaktion des Interaktionspartners mit Analytbindungspartner und Analyt (komplexierendes Verfahren). In Ausführungsformen ist das Verfahren ein kompetitives Verfahren, d. h. Analyt und Interaktionspartner konkurrieren um die Bindung an den Analytbindungspartner. Ein kompetitives Verfahren wird beispielsweise in der DE 10 2020 101 223 A1 beschrieben. Mit zunehmender Konzentration des Analyten in der Probe, nimmt die Bindung des Interaktionspartners und folglich des Partikels an den Analytbindungspartner auf der Sensoroberfläche ab.

In Ausführungsformen ist das Verfahren ein komplexierendes Verfahren, d. h. der Interaktionspartner und Analytbindungspartner binden jeweils an den Analyten. Ein komplexierendes Verfahren wird beispielsweise in der DE 10 2020 124 279 A1 beschrieben. Mit zunehmender Konzentration des Analyten in der Probe, nimmt die Bindung des Interaktionspartners und folglich des Partikels an den Komplex aus Analyten und Analytbindungspartner auf der Sensoroberfläche zu.

In Ausführungsformen ist der Analyt aus der Gruppe, umfassend ein Pestizid, eine hormonell aktive Verbindung, Metallpartikel, Metallion, Virus, bevorzugt Bakteriophagen oder Circoviridae; Bakterium, Biomarker und Antibiotika, ausgewählt. In Ausführungsformen ist der Analyt ein Antibiotikum. In Ausführungsformen ist der Analyt ein Sulfonamid, insbesondere Sulfamethoxazol oder Sulfanilamid. Unter "Sulfonamiden" werden chemische Verbindungen aus der Gruppe der aromatischen Sulfonsäureamide verstanden, welche sich von der 4-Aminobenzolsulfonsäure ableiten.

Vorteilhaft ist mit dem erfindungsgemäßen Verfahren auch ein Nachweis sehr kleiner Analyten, z. B. Metallionen; möglich. Vorteilhaft ist mit dem erfindungsgemäßen Verfahren ein Nachweis von Analyten möglich, deren direkte Immobilisierung aufgrund, z. B. der geringen Größe, der Verfügbarkeit, der Toxizität oder der Pathogenität nicht möglich ist.

In Ausführungsformen ist der Analyt ein Metallpartikel, insbesondere ein Metallnanopartikel, oder ein Metallion, insbesondere ein Schwermetallpartikel oder Schwermetallion.

Unter dem Begriff "Schwermetall" wird ein Metall mit einer Dichte von mindestens 4,5 g/cm³ verstanden, insbesondere Antimon, Arsen, Blei, Cadmium, Chrom, Eisen, Germanium, Gold, Kobalt, Kupfer, Mangan, Molybdän, Nickel, Platin, Polonium, Quecksilber, Rhodium, Selen, Silber, Tantal, Thallium, Titan, Vanadium, Wismut (Bismut), Zink, Zinn oder Zirkon. Nachteilig sind viele Schwermetalle bereits in leichter Überkonzentration für den menschlichen Organismus gesundheitsschädlich oder giftig.

In Ausführungsformen ist der Analyt ein Metallion, insbesondere ein einwertiges oder zweiwertiges Metallion. In Ausführungsformen ist der Analyt Cu (I), Cu (II), Co(II) oder Zn (II).

In Ausführungsformen ist der Analyt ein Virus, bevorzugt ein Bakteriophage, wie M13 und ΦX174; oder Circoviridae.

Unter dem Begriff "Virus" werden infektiöse organische Strukturen umfassend eine Nukleinsäure, insbesondere DNA oder RNA, verstanden. In Ausführungsformen liegt das Virus als Virion, bevorzugt außerhalb von Zellen (extrazellulär), oder als Virus in Form einer Nukleinsäure, bevorzugt innerhalb einer geeigneten Wirtszelle (intrazellulär), vor.

In Ausführungsformen umfasst das Virion eine Nukleinsäure, insbesondere DNA oder RNA, und eine umschließende Protein-Kapsel (Kapsid) oder ein Ribonucleoprotein. In weiteren Ausführungsformen umfasst das Virion eine Lipiddoppelschicht mit viralen Membranproteinen.

Unter dem Begriff "Bakteriophagen" werden Viren verstanden, die auf Bakterien als Wirtszellen spezialisiert sind.

Unter dem Begriff "M13" wird ein filamentöser Bakteriophage bestehend aus einer einzelsträngigen, zirkulären DNA (ssDNA) positiver Polarität verstanden, welcher 6407 Nukleotide umfasst und mit ungefähr 2700 Kopien des *major coat proteins* P8 und 5 Kopien zweier verschiedener *minor coat proteins* (P9, P6, P3) an den Enden verkapselt ist. M13 infiziert *Escherichia coli (E. coli).*

Unter dem Begriff "ΦX174" wird ein Bakteriophage bestehend aus einer einzelsträngigen, zirkulären DNA (ssDNA) verstanden, welcher 5386 Nukleotide umfasst. ΦX174 infiziert *Escherichia coli (E. coli).*

Unter dem Begriff "Circoviridae" wird eine Virenfamilie verstanden, welche einzelsträngige, zirkuläre DNA mit negativer oder ambisense Polarität als Genom und unbehüllte Kapside aufweist.

In Ausführungsformen ist der Analyt ein Biomarker, insbesondere ein protein- und/oder kohlenhydratbasierter Tumormarker oder ein Endotoxin; oder ein Bakterium.

Unter dem Begriff "Biomarker" werden Moleküle verstanden, welche auf Toxine, Pathogene oder Krankheiten hinweisen, insbesondere Bestandteile von Pathogenen, Zellen, Genen oder Genprodukten, bevorzugt Tumormarker oder Endotoxine.

Unter dem Begriff "Endotoxine" werden Lipopolysaccharide verstanden, welche Zerfallsprodukte von Bakterien, insbesondere der äußeren Zellmembran von gramnegativen Bakterien oder Cyanobakterien, sind. Endotoxine können im Menschen und manchen Tierarten gesundheits- bzw. lebensbedrohliche physiologische Reaktionen auslösen.

Unter dem Begriff "Bakterien" werden Prokaryoten verstanden, welche eine die DNA, das Cytoplasma und die Ribosomen umschließende Zellmembran aufweisen, wobei die DNA als dicht gepacktes, in sich geschlossenes Molekül im Cytoplasma frei vorliegt. Weiterhin weisen Bakterien eine RNA-Polymerase bestehend aus 5 Untereinheiten (α (2x), β, β' und ω) auf.

In Ausführungsformen ist der Analyt Glyphosat. In weiteren Ausführungsformen ist der Analyt eine hormonell aktive Verbindung. Unter dem Begriff "hormonell aktive Verbindung" werden Verbindungen verstanden, welche an Östrogenrezeptoren, bevorzugt Estrogenrezeptor-α oder Estrogenrezeptor-β, binden.

In Ausführungsformen ist der Analyt eine Verbindung nach Formel (I) oder (II) 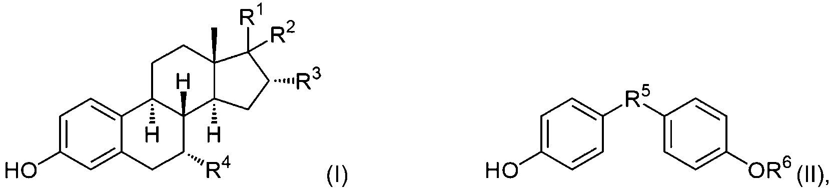
wobei R¹, R², R³, R⁴, R⁶ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen, und
wobei R⁵ ausgewählt ist aus unsubstituierten oder substituierten C1- bis C15-Alkyl-, Alkenyl- oder Arylgruppen oder Kohlenhydratgruppen.

Unter dem Begriff "funktionelle Gruppe" wird eine Atomgruppe in einer Verbindung verstanden, welche das Reaktionsverhalten der Verbindung bestimmt. In Ausführungsformen sind funktionelle Gruppen aus funktionellen Gruppen umfassend Heteroatome (O, N, S, P oder Halogene), insbesondere Carbonsäuren, Halogenide, Thiocarbonsäuren, Sulfonsäuren, Thiolester, Sulfoxide, Carbonsäureanhydride, Carbonsäureester, Carbonsäureamide, Nitrile, Aldehyde, Thioaldehyde, Ketone, Thioketone, Oxime, Hydrazone, Alkohole, Thiole, Amine, Imine, Hydrazine, Ether oder Thioether; und funktionellen Gruppen ohne Heteroatome, insbesondere Doppelbindungen, Dreifachbindungen oder Aromaten, ausgewählt.

In Ausführungsformen ist R², R³, R⁴ und/oder R⁶ H.

In weiteren Ausführungsformen ist R¹ eine Aldehydgruppe, eine Alkoholgruppe oder eine veresterte Alkoholgruppe.

In weiteren Ausführungsformen umfasst R⁵ eine substituierte Ethenylgruppe, eine Aryl- oder eine Heteroarylgruppe.

In Ausführungsformen ist R¹, R² oder R⁶ eine Sulfatgruppe.

In Ausführungsformen ist die Verbindungen nach Formel (II) Bisphenol A .

In alternativen Ausführungsformen ist der Analyt ein Nonylphenol, ein Nonylphenolethoxylat oder ein hydroxylierter (poly-)halogenierter aromatischer Kohlenwasserstoff, insbesondere polychlorierte Dibenzo-p-Dioxine und Dibenzofurane, polybromierte Diphenylether und halogenierte Bisphenol A-Derivate.

Unter dem Begriff "Nonylphenol" werden Verbindungen der allgemeinen Summenformel C₁₅H₂₄O mit einer Molmasse von 220,35 Da verstanden, welche eine Phenolgruppe aufweisen.

Unter dem Begriff "hydroxylierte (poly-)halogenierte aromatische Kohlenwasserstoffe" werden Halogenaromaten verstanden, bei denen mindestens ein Wasserstoffatom durch ein Halogen, insbesondere Fluor, Chlor, Brom und/oder lod, und mindestens ein Wasserstoffatom durch eine Hydroxylgruppe ersetzt ist.

Kester et al. offenbaren hydroxylierte (poly-)halogenierte aromatische Kohlenwasserstoffe (Kester et al. 2002).

In Ausführungsformen ist der Analyt aus Östrogenen, bevorzugt Ethinylestradiol, Estron, Estradiol, Estriol, Equilin oder β-Estradiol-17-(β-D-Glucuronid); Östrogenderivaten, hydroxylierten (poly-)halogenierten aromatischen Kohlenwasserstoffen, Nonylphenolen, Bisphenol A, phenolischen Antiöstrogenen, bevorzugt Diethylstilbestrol, 4-Hydroxytamoxifen, Raloxifen oder Fulvestrant; und phenolischen Phytoöstrogenen, bevorzugt Genistein, Daidzein oder Coumestrol; ausgewählt.

Erfindungsgemäß werden unter dem Begriff "Östrogene" hormonell aktive Verbindungen, insbesondere Steroide, verstanden, welche als Grundgerüst Estran mit einem phenolischen A-Ring aufweisen. Vorteilhaft binden Östrogene an den Östrogenrezeptor.

Unter dem Begriff "Antiöstrogene" werden Verbindungen verstanden, welche an den Östrogenrezeptor ohne Aktivierung binden.

Unter dem Begriff "Phytoöstrogene" werden sekundäre Pflanzenstoffe verstanden, welche an den Östrogenrezeptor binden und daher eine östrogene oder anti-östrogene Wirkung aufweisen.

Zweckmäßig interagiert der Analytbindungspartner mit dem Analyten.

In Ausführungsformen bildet der Interaktionspartner einen Komplex mit Analyten und Analytbindungspartner (komplexierendes Verfahren). Zweckmäßig erfolgt mit der Bindung des Interaktionspartners an den Analyten, welcher an den Analytbindungspartner bindet, eine Interaktion zwischen dem deformierbaren Partikel und der Sensoroberfläche. Mit zunehmender Konzentration des Analyten nimmt die Anzahl der gebildeten Komplexe aus Analyten, Analytbindungspartner und Interaktionspartner zu, sodass die Adhäsionsstärke des Partikels an der Sensoroberfläche erhöht wird.

In Ausführungsformen ist der Analytbindungspartner und der Interaktionspartner jeweils unabhängig voneinander aus der Gruppe, umfassend ein Metall- oder Metallionen-bindendes Peptid oder Protein, einen Chelatbildner, ein Enzym, ein Membranprotein, ein Lipopolysaccharid oder eine Nukleinsäure, insbesondere einer *single-stranded* DNA (ssDNA) oder microRNA (miRNA) ausgewählt. In weiteren Ausführungsformen sind der Analytbindungspartner und der Interaktionspartner verschieden. Vorteilhaft kann durch Einsatz verschiedener Analytbindungspartner und Interaktionspartner die Selektivität des Nachweises erhöht werden, da durch die Verwendung eines Analytbindungspartners und eines von diesem verschiedenen Interaktionspartners, welche beide denselben Analyten binden, die Wahrscheinlichkeit für falschpositive Ergebnisse aufgrund unspezifischer Bindung verringert wird.

In Ausführungsformen sind der Analytbindungspartner und der Interaktionspartner identisch.

Vorteilhaft ermöglichen identische Analytbindungspartner und Interaktionspartner, ausgewählt aus Metall- oder Metallionen-bindendes Peptid oder Protein, einen Chelatbildner, ein Enzym, ein Membranprotein, ein Lipopolysaccharid oder eine Nukleinsäure, die gleichzeitige Interaktion des Analyten mit dem Analytbindungspartner und dem Interaktionspartner.

In Ausführungsformen interagiert der Interaktionspartner mit dem Analytbindungspartner (kompetitives Verfahren). Zweckmäßig erfolgt mit der Interaktion des Interaktionspartners mit dem Analytbindungspartner eine Interaktion zwischen dem deformierbaren Partikel und der Sensoroberfläche. Zweckmäßig konkurrieren Interaktionspartner und Analyt um die Bindung an den Analytbindungspartner. Mit zunehmender Konzentration des Analyten nimmt die Bindung von Interaktionspartnern an den Analytbindungspartner und die Bindung des Partikels an die Sensoroberfläche ab.

In Ausführungsformen sind der Analyt und der Interaktionspartner identisch.

Unter dem Begriff "Metall- oder Metallionen-bindenden Peptid oder Protein" werden Peptide oder Proteine verstanden, welche anziehende Wechselwirkungen (elektrostatische Wechselwirkungen, Van-der-Waals-Wechselwirkungen, hydrophobe Wechselwirkungen) mit Metallen oder Metallionen eingehen. Metallionen sind positiv geladene Ionen von Metallen.

In Ausführungsformen ist das Metallionen-bindende Protein ein Metallionen-bindendes Transportprotein. In Ausführungsformen werden Metall-bindende oder Metallionen-bindende Peptide oder Proteine mittels Phagendisplay bereitgestellt.

Unter dem Begriff "Chelatbildner" werden chemische Verbindungen verstanden, welche mindestens zwei koordinative Bindungen mit einem Metallion oder Metallatom bilden.

In Ausführungsformen ist der Chelatbildner aus Diethylentriaminpentaessigsäure (DTPA), 1,4,7,10-Tetraazacyclododecan-N,N',N",N"'-tetraessigsäure (DOTA), Mercaptoacetyltriglycin (MAG3), 6-Hydrazinopridin-3-carboxylsäure (Hynic), Hydroxybenzylethylendiamin (HBED), N,N'-Bis [2-hydroxy-5-(carboxyethyl)benzyl]ethylenediamin-N,N'-diessigsäure (HBED-CC) oder 2-(3-(1-Carboxy-5-[(6-fluoro-pyridin-3-carbonyl)-amino]-pentyl)-ureido)-pentandicarbonsäure (DCFPyL) ausgewählt.

Unter dem Begriff "Membranprotein" werden Proteine verstanden, welche mit der Zellmembran oder einer Biomembran von Zellkompartimenten bzw. Organellen einer Zelle assoziiert sind. In Ausführungsbeispielen sind Membranproteine periphere Membranproteine (auch Oberflächenproteine), welche an die Membranoberfläche gebunden sind, oder integrale Membranproteine, welche mit einem hydrophoben Anteil in die Doppellipidschicht der Membran integriert sind oder die Membran als Transmembranprotein durchspannen.

Unter dem Begriff "Lipopolysaccharide (LPS)" werden Moleküle verstanden, welche aus verschiedenen Regionen bestehen. Der Bereich, welcher für die Verankerung der LPS an den hydrophoben Bereich der bakteriellen äußeren Membran dient, ist Lipid A, bestehend meist aus N-Acetylglucosamin mit gebundenen Fettsäuren. Der Polysaccharidanteil enthält ein "Kern"-Polysaccharid sowie einen Bereich mit sich wiederholenden Zuckeranteilen.

Unter dem Begriff "Nukleinsäure" werden aus Nukleotiden aufgebaute Makromoleküle mit einem Zucker-Phosphat-Rückgrat verstanden. In Ausführungsformen ist die Nukleinsäure eine Ribonukleinsäure (RNA) oder eine Desoxyribonukleinsäure (DNA).

Zweckmäßig umfassen die Peptide, Proteine, Lipopolysaccharid und Nukleinsäuren auch deren Derivate. Unter dem Begriff "Derivat" wird ein Molekül verstanden, insbesondere ein metallionbindendes Peptid, ein Membranprotein, ein Lipopolysaccharid oder eine Nukleinsäure; mit einem hohen Grad an struktureller Identität mit dem Molekül, vorzugsweise dem gleichen Gerüst, wobei mindestens ein Atom, eine Gruppe von Atomen, eine funktionelle Gruppe oder eine Substruktur durch ein anderes Atom, eine Gruppe von Atomen, eine funktionelle Gruppe oder eine Substruktur, z.B. eine Alkylgruppe, insbesondere Methylgruppe; eine Glucuronsäuregruppe, ein Halogen, eine Hydroxylgruppe oder eine Sulfatgruppe, ersetzt ist. Vorteilhaft interagieren Derivate mit dem Analyten.

Zweckmäßig umfassen Derivate weiterhin Peptide oder Proteine mit einer Sequenzidentität von mindestens 90 %, bevorzugt 95 %, besonders bevorzugt 99 %, wobei der Unterschied in der Aminosäuresequenz von maximal 10 %, bevorzugt maximal 5 %, besonders bevorzugt maximal 1 % insbesondere eine Verkürzung der Aminosäuresequenz, eine Verlängerung der Aminosäuresequenz und/oder ein Austausch einzelner Aminosäuren ist. Bevorzugt weisen die Peptidderivate oder Proteinderivate eine Sequenzidentität von mindestens 90 %, bevorzugt 95 %, besonders bevorzugt 99 %, außerhalb der Bindestellen des Analytbindungspartners bzw. Interaktionspartners für den Analyten auf und eine Sequenzidentität der Bindestellen des Analytbindungspartners bzw. Interaktionspartners für den Analyten von 100 %.

In Ausführungsformen umfassen Peptid- oder Proteinderivate D-Aminosäuren, Pseudopeptidbindungen, Aminoalkohole, nicht-proteinogene Aminosäuren, Aminosäuren mit modifizierten Seitenketten und/oder zirkuläre Peptide.

In Ausführungsformen sind Nukleinsäurederivate aus Peptidnukleinsäuren, wie γ-Peptidnukleinsäuren ausgewählt.

In Ausführungsformen ist der Interaktionspartner aus Glyphosat, Phosphoenolpyruvat, Phosametin oder einer Verbindung nach Formel (I) oder (II) 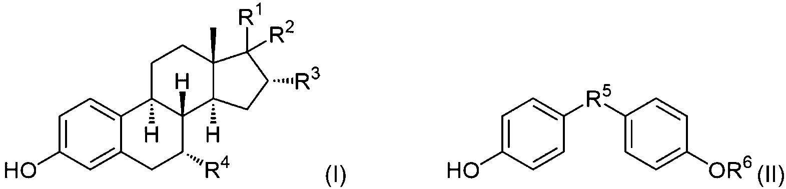 ausgewählt,
wobei R¹, R², R³, R⁴, R⁶ jeweils unabhängig voneinander aus H, funktionellen Gruppen, unsubstituierten oder substituierten C1- bis C15-Alkyl-, Alkenyl- oder Arylgruppen oder Kohlenhydratgruppen ausgewählt ist,
wobei R⁵ aus unsubstituierten oder substituierten C1- bis C15-Alkyl-, Alkenyl- oder Arylgruppen oder Kohlenhydratgruppen ausgewählt ist,
wobei der Interaktionspartner eine funktionelle Gruppe in Position R¹, R² oder R⁶ aufweist.

In Ausführungsformen ist der Analyt Glyphosat und der Analytbindungspartner 5-Enolpyruvylshikimat-3-phosphat-Synthase.

In Ausführungsformen ist der Analyt eine hormonell aktive Verbindung und der Analytbindungspartner eine Sulfotransferase.

In Ausführungsformen ist der Analyt Cu (I) und der Analytbindungspartner und optional der Interaktionspartner abhängig voneinander aus Cox17 und Sco1 ausgewählt. In einer Ausführungsform umfasst der Analytbindungspartner Cox17 und der Interaktionspartner Sco1, bevorzugt eine trunkierte Mutante von Sco1 ohne Transmembrandomäne. In einer weiteren Ausführungsform umfasst der Analytbindungspartner Sco1 und der Interaktionspartner Cox17.

Unter dem Begriff "Sco1" wird ein Chaperon der Cytochrom C-Oxidase in der mitochondrialen Atmungskette verstanden.

Unter dem Begriff "Cox17" wird ein Kupfer-Transport-Protein verstanden.

In Ausführungsformen ist der Analyt ein Virus und der Analytbindungspartner und optional der Interaktionspartner jeweils unabhängig voneinander aus einem Protein nach Letarov und Kulikov (Letarov und Kulikov 2017), insbesondere einem Rezeptor ausgewählt aus der Gruppe umfassend OmpA, OmpC, OmpF, O157-Antigen, Lipopolysaccharid (LPS), LPS-O-Antigen, O-Methylphosphoramidate (MeOPN), Flagellum, FliC, FljB, FliK, GamR (LPXTG-enthaltendes Protein), CsaB, TolC, BtuB, LamB, TonA, TonB, FhuA, Tsx, RsaA130K-Protein des S-Layer, Sap-Protein des S-Layer, SlpH-Protein des S-Layer, Teichonsäure des Zellwand, glykosylierte Poly(Gro-P)-Teichonsäure der Zellwand, Serovar-spezifische Teichonsäure der Zellwand, Lipoteichonsäure der Zellwand (LTA), Rhamnosereste in Teichonsäure der Zellwand.

In Ausführungsformen ist der Analyt ein Biomarker oder ein Bakterium und der Analytbindungspartner und optional der Interaktionspartner jeweils unabhängig voneinander aus einem Peptid, einem Protein, einem Enzym, einer Nukleinsäure oder deren Derivaten ausgewählt.

In Ausführungsformen weist der Analytbindungspartner und optional der Interaktionspartner das aktive Zentrum oder die Substrat-Bindedomäne eines Enzyms, z. B. ein Sushi-Peptid, auf. Unter dem Begriff "Sushi-Peptid" wird eine Endotoxin-Bindestelle von Faktor C, einer Lipopolysaccharid-sensitiven Serinprotease des Pfeilschwanzkrebses, verstanden. In Ausführungsformen ist der Analytbindungspartner und/oder der Interaktionspartner das Sushi-Peptid S1 und/oder S3.

In Ausführungsformen ist der Analyt ein Antibiotikum, bevorzugt ein Sulfonamid, insbesondere Sulfamethoxazol oder Sulfanilamid, und der Analytbindungspartner ein Enzym, ein aktives Zentrum oder eine Substrat-Bindedomäne eines Enzyms ist, bevorzugt eine Dihydropteroat-Synthase (DHPS).

In Ausführungsformen der Erfindung ist die Oberfläche transparent, semitransparent oder opak ausgebildet. In weiteren Ausführungsformen ist die Oberfläche zumindest in einem Bereich von 400 nm bis 700 nm, bevorzugt im Bereich von 500 nm bis 600 nm, transparent ausgebildet.

In Ausführungsformen der Erfindung ist die Oberfläche planar ausgebildet. Beispielsweise kann die Oberfläche ein Glas- oder Kunststoffformkörper sein, z. B. ein Objektträger, Deckgläschen, Siliziumwafer oder ähnliches. In Ausführungsformen der Erfindung ist die Oberfläche als Multi-Well-Platte, insbesondere 24 Well-Platte ausgebildet.

In Ausführungsformen liegt die Länge der Sensoroberfläche im Bereich von 1 mm bis 1 cm, bevorzugt 2 mm bis 5 mm. Unter der Länge der Sensoroberfläche wird die Dimension der Sensoroberfläche verstanden entlang der in Schritt f) die Bewegung der Partikel bestimmt wird.

In Ausführungsformen erfolgt die Bereitstellung von mindestens zwei Sensoroberflächen, bevorzugt zwei bis 24 Sensoroberflächen, besonders bevorzugt zwei bis sechs Sensoroberflächen; umfassend jeweils einen immobilisierten Analytbindungspartner.

Zweckmäßig können die mindestens zwei Sensoroberflächen auf einem Sensor (Formkörper) als mindestens zwei verschiedene lokal begrenzte Teilbereiche oder zwei unterschiedliche Sensoren (Formkörper) ausgebildet sein. Zweckmäßig sind die mindestens zwei Sensoroberflächen parallel angeordnet, d. h. die Dimension der Sensoroberflächen, entlang derer die Bewegung des deformierbaren Partikels erfolgt, sind parallel zueinander angeordnet.

In Ausführungsformen erfolgt der Nachweis mindestens eines Analyten in einer Probe auf jeweils einer Sensoroberfläche.

In Ausführungsformen ist der immobilisierte Analytbindungspartner auf der ersten Sensoroberfläche und der immobilisierte Analytbindungspartner auf der mindestens zweiten Sensoroberfläche gleich oder unterschiedlich.

Vorteilhaft kann durch die Bereitstellung von mindestens zwei Sensoroberflächen mit gleichen Analytbindungspartnern eine parallele Messung einer Probe in unterschiedlichen Konzentrationen oder im Vergleich zu einer entsprechenden Kontrollproben erfolgen. Unter dem Begriff "parallele Messung" wird eine Durchführung des Verfahrens zum Nachweis eines Analyten mit mindestens zwei Sensoroberflächen nahezu zeitgleich verstanden, wobei eine simultane Detektion des Analyten erfolgt.

In Ausführungsformen erfolgt eine parallele Messung von mindestens zwei unterschiedlichen Konzentrationen einer Probe auf mindestens zwei Sensoroberflächen mit gleichen Analytbindungspartnern.

In Ausführungsformen erfolgt eine parallele Messung von mindestens einer Probe, eine Negativkontrolle, d. h. einer Probe ohne Analyten, bevorzugt mit gleicher Matrix; einer Positivkontrolle, d. h. einer Probe mit Analyten, bevorzugt mit gleicher Matrix; und/oder einer Kalibrierreihe.

In Ausführungsformen erfolgt eine parallele Messung von mindestens einer Probe auf mindestens zwei Sensoroberflächen mit unterschiedlichen Dichten des immobilisierten Analytbindungspartners. Vorteilhaft wird durch die Messung einer Probe auf mindestens zwei Oberflächen mit zwei verschiedenen Dichten des Analytbindungspartners die Genauigkeit des erfindungsgemäßen Verfahrens zur Konzentrationsbestimmung des Analyten erhöht.

Die Funktionalisierung der Sensoroberfläche durch Immobilisierung eines Analytbindungspartners bestimmt die Interaktion mit dem Analyten und dem an das deformierbare Partikel gebundenen Interaktionspartner. Zweckmäßig erfolgt mit Zunahme der Kopplungsdichte und/oder Verbesserung der Zugänglichkeit des Analytbindungspartners eine Erhöhung der maximal nachweisbaren Analytkonzentration. Daher kann über eine Variation der Funktionalisierung der Sensoroberfläche, insbesondere der Kopplungsdichte und Zugänglichkeit (Orientierung) von für die Interaktion entscheidenden Bindungsstellen des Analytbindungspartners, diese Interaktion und damit die Bindungsstärke der Partikel an die Sensoroberfläche in Abhängigkeit der Analytkonzentration und damit eine mögliche Bewegung beeinflusst werden.

In Ausführungsformen erfolgt die Immobilisierung des Analytbindungspartners an der Sensoroberfläche über eine Proteindomäne, ausgewählt aus Hydrophobinen, ECM-Proteinen, S-Layer Proteinen, Peptidlinkern und Protein-Tags, insbesondere His-Tag; oder durch einen Polymerlinker, z. B. Polyethylenglykol (PEG). Dadurch wird eine gerichtete Immobilisierung an die Oberfläche ermöglicht, d. h. die Bindungsstelle des Interaktionspartners oder Analyten am Analytbindungspartner ist zugänglich auf der Oberfläche orientiert.

Unter dem Begriff "immobilisiert" wird eine irreversible oder pseudoirreversible Bindung umfassend kovalente, koordinative, metallische, ionische Bindungen, Wasserstoffbrückenbindungen und/oder Van-der-Waals-Wechselwirkungen verstanden.

In weiteren Ausführungsformen erfolgt die Immobilisierung durch kovalente Bindung über eine funktionelle Gruppe des Analytbindungspartners. In Ausführungsformen erfolgt eine gerichtete oder eine ungerichtete Immobilisierung des Analytbindungspartners an der Oberfläche. Bei der ungerichteten Immobilisierung kann teilweise die Bindungsstelle des Interaktionspartners oder Analyten am Analytbindungspartner blockiert sein, sodass zufällig verteilt nicht alle Analytbindungspartner für die Interaktion zur Verfügung stehen.

Unter dem Begriff "funktionelle Gruppe" wird eine Atomgruppe in einer Verbindung verstanden, welche das Reaktionsverhalten der Verbindung bestimmt. In Ausführungsformen sind funktionelle Gruppen aus funktionellen Gruppen umfassend Heteroatome (O, N, S, P oder Halogene), insbesondere Carbonsäuren, Halogenide, Thiocarbonsäuren, Sulfonsäuren, Thiolester, Sulfoxide, Carbonsäureanhydride, Carbonsäureester, Carbonsäureamide, Nitrile, Aldehyde, Thioaldehyde, Ketone, Thioketone, Oxime, Hydrazone, Alkohole, Thiole, Amine, Imine, Hydrazine, Ether oder Thioether; und funktionellen Gruppen ohne Heteroatome, insbesondere Doppelbindungen, Dreifachbindungen oder Aromaten, ausgewählt.

In weiteren Ausführungsformen ist der Analytbindungspartner ein Fusionsprotein. Dabei weist das Fusionsprotein mindestens zwei Proteindomänen auf, welche unterschiedliche Funktionalitäten aufweisen, insbesondere eine Analytbindungsstelle und eine Proteindomäne, ausgewählt aus Hydrophobinen, ECM (*extracellular matrix*)-Proteinen, S-Layer-Proteinen, Peptidlinkern und Tags. Die Analytbindungsstelle dient der Anbindung des Analyten an den Analytbindungspartner. Unter dem Begriff "Tag" (auch Protein-Tag, Affinitäts-Tag oder Epitop-Tag) wird ein Marker, insbesondere eine Proteinsequenz oder ein organisches Molekül, verstanden, der an den Analytbindungspartner (Protein) gebunden ist, um dessen Anbindung an die Sensoroberfläche zu ermöglichen.

In Ausführungsformen sind Tags organische Moleküle einschließlich Fluoreszenzmarkierungen, z. B. FITC (Fluoresceinisothiocyanat), und Biotin. In Ausführungsformen sind Tags Peptid-Tags, insbesondere His-Tag, GST (Glutathion-S-Transferase)-Tag oder Spy-Tag. Zweckmäßig erfolgt die Immobilisierung des Analytbindungspartners mittels Tags an der Oberfläche über Ni-NTA (nickelbeladenes Affinitätsharz)-His-Tag-Kopplung, Glutathion-GST-Tag-Kopplung oder SpyCatcher-SpyTag-Kopplung.

Bevorzugt ist der Analytbindungspartner ein Fusionsprotein mit einem Protein-Tag oder Hydrophobin.

In Ausführungsformen erfolgt die Immobilisierung des Analytbindungspartners an der Oberfläche aus einer Lösung, insbesondere einer wässrigen Lösung.

In Ausführungsformen ist die Probe eine wässrige Lösung oder eine wässrige Dispersion. Unter dem Begriff "Dispersion" (auch disperse Flüssigkeit) wird ein heterogenes Stoffgemisch verstanden, bei dem mindestens eine feste oder flüssige Verbindung in einer Lösung verteilt ist. In Ausführungsformen ist die Dispersion eine Suspension oder Emulsion.

Bevorzugt ist die Probe eine wässrige Lösung.

Zweckmäßig werden der pH-Wert und die Salzkonzentration der Probe entsprechend den Interaktionseigenschaften von Analyten, Analytbindungspartner und Interaktionspartner vor der Kontaktierung der Probe mit der Sensoroberfläche eingestellt.

In Ausführungsformen weist die wässrige Lösung eine Salzkonzentration im Bereich von 0 mmol/I bis 300 mmol/I auf.

In Ausführungsformen ist die wässrige Lösung eine isotonische Salzlösung. Unter dem Begriff "isotonische Salzlösung" wird eine Salzlösung verstanden, welche denselben osmotischen Druck wie das menschliche Blut aufweist.

In Ausführungsformen ist die Probe aus folgenden Proben ausgewählt: Körperflüssigkeiten, wie Speichelproben, Blutproben, Blutplasmaproben, Blutserumproben, Urinproben oder Schleimproben; Gewässerproben, Trinkwasserproben, Abwasserproben, Proben aus chemischen Produktionsprozessen, extrahierte Bodenproben, Lebensmittelproben, Futtermittelproben, Proben aus biologischen und biochemischen Produktionsprozessen, Fermentationslösungen, Waschmittelproben, Spülmittelproben, Körperpflegemittelproben, Kosmetikproben oder Pharmakaproben. In Ausführungsformen werden verdünnte Proben der vorgenannten Art oder verdünnte Extrakte von z. B. Bodenproben, Schleimproben, Fermentationslösungen und dergleichen verwendet.

Zweckmäßig ist die wässrige Lösung frei von Schwebstoffen. In Ausführungsformen erfolgt eine Filtration der wässrigen Lösung vor der Durchführung des erfindungsgemäßen Verfahrens, insbesondere vor Schritt c).

Das Verfahren kann bei einer Temperatur durchgeführt werden, die das Einfrieren und Denaturieren des Analyten, des Analytbindungspartners sowie Interaktionspartners vermeidet.

In Ausführungsformen liegt die Temperatur im Bereich von 0 °C bis 100 °C, bevorzugt im Bereich von 0 °C bis 50 °C, besonders bevorzugt im Bereich von 10 °C bis 30 °C.

Erfindungsgemäß erfolgt in Schritt d) das Kontaktieren der Sensoroberfläche mit einem deformierbaren Partikel, wobei das deformierbare Partikel mit einem Interaktionspartner modifiziert ist, sodass in Abhängigkeit der Konzentration des Analyten eine Interaktion zwischen Partikel und Sensoroberfläche erfolgt.

Dabei erfolgt die Bindung zwischen deformierbaren Partikeln und Sensoroberfläche in Abhängigkeit vom Analyten, Analytbindungspartner und Interaktionspartner, insbesondere in Abhängigkeit der Interaktion von Analytbindungspartner und Interaktionspartner, welche von der Analytkonzentration abhängig ist.

In Ausführungsformen erfolgen die Schritte a), c) und d) mit einer Ausrichtung der Sensoroberfläche in horizontaler Lage.

In Ausführungsformen erfolgt Schritt d) mit einer Ausrichtung der Sensoroberfläche in horizontaler Lage.

In Ausführungsformen erfolgt Schritt d) für einen Zeitraum im Bereich von 1 min bis 5 min.

In Ausführungsformen ist das deformierbare Partikel ein Hydrogelpartikel, bevorzugt ein Poly(ethylenglykol)hydrogelpartikel. Vorteilhaft wird eine Stabilität der Hydrogelpartikel für mindestens 28 Tage bei 4 °C beobachtet.

In Ausführungsformen weist das deformierbare Partikel ein Elastizitätsmodul im Bereich von 5 kPa bis 100 kPa auf. Das Elastizitätsmodul des deformierbaren Partikels kann aus Kraft-Abstands-Kurven der Rasterkraftspektroskopie (SFM) bestimmt werden.

Zweckmäßig kann durch das Elastizitätsmodul die Bindung des mit dem Interaktionspartner modifizierten Partikels an die Sensoroberfläche eingestellt werden. Zweckmäßig bewirkt ein geringeres Elastizitätsmodul eine bessere Deformierbarkeit des Partikels bei Interaktion mit der Sensoroberfläche und führt dabei zu einer höheren Bindungsstärke durch die Ausbildung von mehr Interaktionen von Analytbindungspartner und Interaktionspartner oder Analytbindungspartner, Analyt und Interaktionspartner. Dementsprechend ergeben sich geringere Grenzwerte der Analytkonzentration für eine Bewegung der Partikel entlang der Sensoroberfläche.

In Ausführungsformen weist das deformierbare Partikel einen Durchmesser im Bereich von 10 µm bis 100 µm auf, bevorzugt einen Durchmesser von etwa 25 µm auf. Der Durchmesser des deformierbaren Partikels kann durch optische Hellfeldmikroskopie bestimmt werden.

Zweckmäßig kann über den Durchmesser der Partikel die Sedimentationskraft eingestellt werden. Im Allgemeinen bewirkt ein geringerer Durchmesser eine geringere Sedimentationskraft der Partikel. Dementsprechend ergeben sich für kleinere Partikel geringere Grenzwerte der Analytkonzentration für eine Bewegung der Partikel auf der Sensoroberfläche.

In Ausführungsformen ist das deformierbare Partikel farbig oder weist eine farbige Markierung auf.

In Ausführungsformen weist das deformierbare Partikel eine Markierung mit mindestens einem absorbierenden Farbstoffmolekül oder einem absorbierenden Nanopartikel (z.B. Gold) auf. Zweckmäßig erfolgt die Markierung ohne eine signifikante Veränderung der mechanischen Eigenschaften und der Oberflächeneigenschaften des Partikels.

Unter dem Begriff "Modifizierung" wird eine Veränderung des deformierbaren Partikels verstanden, bei der ein Interaktionspartner an das deformierbare Partikel kovalent gebunden wird.

In Ausführungsformen ist der Interaktionspartner über eine funktionelle Gruppe und/oder über einen Linker an das deformierbare Partikel gebunden.

In Ausführungsformen ist das deformierbare Partikel ein funktionalisiertes Partikel, wobei die Oberfläche des deformierbaren Partikels eine Funktionalisierung, insbesondere funktionelle Gruppen wie Carboxyl-, Thiol-, Maleimid- oder Aminogruppen, aufweist. Die Funktionalisierung dient dabei der Immobilisierung des Interaktionspartners.

Die Modifizierung des deformierbaren Partikels mit dem Interaktionspartner bestimmt die Interaktion mit dem an die Sensoroberfläche gebundenem Analytbindungspartner oder Analyt. Dementsprechend bestimmt die Kopplungsdichte und Zugänglichkeit von für die Interaktion entscheidenden Bindungsstellen des Interaktionspartners diese Interaktion und damit die Bindungsstärke der Partikel an die Sensoroberfläche oder den Analyten. Somit kann durch die Modifizierung der Partikel die relative Bindungsstärke von Partikel an die Sensoroberfläche in Abhängigkeit der Analytkonzentration und damit eine mögliche Bewegung bei bestimmten Analytgrenzwerten eingestellt werden.

In Ausführungsformen erfolgt die Synthese der Partikel mikrofluidisch, insbesondere nach dem Verfahren gemäß Rettke et al. (Rettke et al. 2022).

In Ausführungsformen erfolgt die Synthese unter Nutzung von Thiol- und Vinylsulfon-funktionalisierten 4-Arm-Polyethylenglykol-Molekülen. In Ausführungsformen erfolgt die Synthese unter Nutzung von Thiol- und Maleimid-funktionalisierten 4-Arm-Polyethylenglykol-Molekülen. In Ausführungsformen erfolgt die Synthese unter Nutzung einer Michael-Addition der Thiol- und Maleimid- oder Vinylsulfon-funktionalisierten 4-Arm-Polyethylenglykol-Moleküle.

In Ausführungsformen erfolgt die Anbringung des Linkers an die mit Thiol-Gruppen funktionalisierten Partikel. In Ausführungsformen erfolgt die Anbringung eines Maleimid-PEG-Succinimidylester-Linkers.

In Ausführungsformen erfolgt die Synthese und Carboxy-Funktionalisierung der Partikel mikrofluidisch mittels photoinitiierter radikalischer Vernetzung von Polyethylenglykol-Diacrylamid oder Polyethylenglykol-Diacrylat mit einem Molekulargewicht im Bereich von 500 Da bis 8.000 Da, bevorzugt etwa 4.000 Da. Vorteilhaft werden monodisperse Partikel mit einem Durchmesser im Bereich von 10 µm bis 100 µm erzeugt. In Ausführungsformen erfolgt eine photoinitiierte radikalische Pfropfung von Acrylsäuremonomeren zur Einführung der Carboxylgruppen.

In Ausführungsformen erfolgt die Synthese und Carboxy-Funktionalisierung der deformierbaren Partikel, insbesondere der Hydrogel-Mikropartikel via Emulsions- und radikalischer Fällungspolymerisation von Polyethylenglykol-Diacrylamid oder Polyethylenglykol-Diacrylat. In Ausführungsformen erfolgt eine radikalische Pfropfung von Acrylsäuremonomeren, Crotonsäuremonomeren oder weiteren Alkenderivaten mit funktionellen Gruppen, wie etwa Aminen, zur Einführung der Carboxyl-, Amino- oder anderen funktionellen Gruppen.

In Ausführungsformen weist der Linker eine Konturlänge im Bereich von 5 Å bis 200 Å, bevorzugt im Bereich 10 Å bis 65 Å; auf. In Ausführungsformen weist der Linker einen Polymerisationsgrad im Bereich von 1 bis 70, bevorzugt im Bereich 3 bis 20; auf. Vorteilhaft ermöglicht ein Linkermolekül die geeignete Immobilisierung des Interaktionspartners über eine funktionelle Gruppe, wobei die Kopplungsgruppe die Funktionalität und Sensitivität des Verfahrens beeinflusst.

Weiterhin vorteilhaft kann über die Länge bzw. den Polymerisationsgrad des Linkers die resultierende Affinität des immobilisierten Interaktionspartners variiert und somit der Arbeitsbereich des Verfahrens eingestellt werden.

In Ausführungsformen ist der Linker ausgewählt aus den Gruppen der homo- und heterobifunktionalen Linker. In Ausführungsformen umfasst der Linker Ethylendiamin, Oligoethylenglykoldiamine, Polyethylenglykoldiamine, Peptide wie Pentaglycin oder Aminosäuren. In Ausführungsformen umfasst der Linker funktionelle Gruppen, wie Thiol- oder Azidgruppen. In Ausführungsformen ist der Linker ein Polyethylenglykoldiamin-Linker.

In Ausführungsformen enthält der Linker Schutzgruppen. In Ausführungsformen ist die Schutzgruppe aus Fluorenylmethoxycarbonyl-, tert-Butyloxycarbonyl- oder tert-Butyl-Schutzgruppen ausgewählt. Vorteilhaft stellen die Schutzgruppen sicher, dass keine unerwünschte Polymerisierung der Linker-Moleküle oder Quervernetzung der deformierbaren Partikel auftritt.

In Ausführungsformen ist die Sensoroberfläche in Schritt e) in einem Winkel im Bereich von 30° bis 150°, bevorzugt 45° bis 135°, besonders bevorzugt von etwa 90°, ausgehend von der horizontalen Achse der Sensoroberfläche geneigt. Unter etwa 90° wird eine Neigung im Bereich von 70° bis 110° verstanden. Erfinderseits wurde erkannt, dass schon eine Neigung ab 30° zur Bewegung der Partikel entlang der Sensorfläche ausreicht. Neben der Neigung ist die Auslösung der Bewegung der Partikel von der Funktionalisierung der Sensoroberfläche und der Modifizierung des Partikels sowie dessen Durchmesser und Elastizitätsmodul abhängig. Bei der Neigung der Sensoroberfläche wurde erkannt, dass der exakte Winkel nicht entscheidend ist, da die Neigung der Sensoroberfläche (im Bereich von ungefähr 90°) nur der Auslösung der Bewegung von nicht oder nur sehr schwach interagierenden Partikeln dient.

In Ausführungsformen erfolgt Schritt e) für einen Zeitraum von mindestens 1 min.

Erfindungsgemäß erfolgt die Detektion der Analyt-abhängigen Bindung der deformierbaren Partikel durch Bestimmung der Bewegung des deformierbaren Partikels entlang der Sensoroberfläche. Unter der Bewegung entlang der Sensoroberfläche wird eine Änderung der Position des deformierbaren Partikels in Richtung der geneigten Sensoroberfläche verstanden. Zweckmäßig erfolgt keine Bewegung, auch bei einer Neigung der Sensoroberfläche von 90°, wenn die Adhäsionskraft des deformierbaren Partikels an der Sensoroberfläche größer als dessen Sedimentationskraft ist. Dieses Verhältnis ist von der Analytkonzentration abhängig, sodass die Bewegung des Partikels zur Konzentrationsbestimmung des Analyten genutzt werden kann.

Das Verhältnis von Adhäsions- und Sedimentationskraft, d. h. die Bindung der Partikel an der Sensoroberfläche, kann über die Funktionalisierung der Sensoroberfläche und des Partikels, dessen Durchmesser und/oder E-Modul angepasst werden. Anhand dieser Parameter kann zum einen die Partikel-Oberflächen-Interaktion, zum anderen die mit dieser Interaktion konkurrierende Sedimentationskraft eingestellt werden, wodurch unterschiedliche Grenzwerte der Analyt-Konzentration nachweisbar sind.

In Ausführungsformen ist das Verfahren ein kompetitives Verfahren, wobei Analyt und Interaktionspartner (gekoppelt an den deformierbaren Partikel) um die Bindung an den Analytbindungspartner konkurrieren, wobei die Bindung des deformierbaren Partikels an den Analytbindungspartner auf der Sensoroberfläche mit zunehmender Konzentration des Analyten abnimmt und sich somit die Wahrscheinlichkeit einer Bewegung des deformierbaren Partikels entlang der Sensoroberfläche erhöht, da dieser nicht stark genug gegenüber der Sedimentationskraft bindet.

In Ausführungsformen ist das Verfahren ein komplexierendes Verfahren, wobei der Interaktionspartner (gekoppelt an den deformierbaren Partikel) den Analyten bindet, welcher den Analytbindungspartner bindet, und somit ein Komplex aus Interaktionspartner mit Analyten und Analytbindungspartner gebildet wird, wobei die Bindung des deformierbaren Partikels mit zunehmender Konzentration des Analyten zunimmt und sich somit die Wahrscheinlichkeit einer Bewegung des deformierbaren Partikels entlang der Sensoroberfläche verringert, da die Bindung des Partikels größer als die Sedimentationskraft ist.

In Ausführungsformen erfolgt die optische Bestimmung der Bewegung des deformierbaren Partikels in Schritt f) visuell, mittels Durchlichtmikroskopie, insbesondere Hellfeldmikroskopie, Phasenkontrastmikroskopie, Holographie-Mikroskopie; oder Streulichtmessung. Vorteilhafterweise ermöglichen die vorgenannten Detektionsmethoden eine einfache, kostengünstige und schnelle Bestimmung der Analytkonzentration, sodass deren Einsatz insbesondere für mobile Anwendungen geeignet ist.

Unter einer optischen Bestimmung versteht man ein Messverfahren, bei dem mit Hilfe von Licht physikalische Größen bestimmt werden. In Ausführungsformen erfolgt eine Bestimmung über den Vergleich zweier zeitversetzter, mikroskopischer Abbildungen bezüglich der Anwesenheit und/oder Position von Partikeln im Beobachtungsfeld. Über die differenzielle Bestimmung der Anzahl der bewegten oder nicht-bewegten Partikel wird der Anteil gebundener Partikel und damit die Stärke der Adhäsionskraft und korrelierten Analytkonzentration bestimmt.

In Ausführungsformen erfolgt eine Korrelation mit Standardproben (Kalibrierung) und/oder einem mit dem erfindungsgemäßen Verfahren gemessenen Grenzwert der Analytkonzentration, d.h. einem maximal zulässigen Wert für die Konzentration des Analyten in einer Probe. In mikroskopischen Verfahren werden die genaue Anzahl von Partikeln und genaue Verhältnisse von gebundenen und nicht-gebundenen Partikeln ermittelt. In visuellen Verfahren werden mittlere Helligkeits-/Farbwerte (optische Dichte) bestimmt.

In Ausführungsformen weist die Sensoroberfläche mindestens einen lokal definierten Bereich für die Detektion (Auge, Kamera, Mikroskop) auf. In Ausführungsformen wird der lokal definierte Bereich konstruktiv durch die Abmessungen der Sensoroberfläche und/oder die Haltevorrichtung im Mikroskop eingestellt oder im visuellen Verfahren (Detektion per Auge) durch Markierung des Messbereichs gewährleistet.

In Ausführungsformen erfolgt die Detektion in Schritt f) durch visuelle Kontrolle der Anwesenheit in einem Sensorfeld, insbesondere unter Verwendung von farbigen oder farbig-markierten Partikeln. Vorteilhaft kann durch eine visuelle Bestimmung unter der Verwendung von farbigen oder farbig-markierten Partikeln einfach, kostengünstig, schnell und ohne technische Hilfsmittel ein Grenzwert der Analytkonzentration bestimmt werden.

In Ausführungsformen erfolgt die Detektion in Schritt f) durch eine Aufnahme von mindestens zwei mikroskopischen Bildern (Durchlichtmikroskopie), die beispielweise in einem zeitlichen Abstand von 30 Sekunden bis 1 Minute aufgenommen werden. Unter Verwendung mikroskopischer Detektionsmethoden ist eine Aussage über die Analytkonzentration nach der Neigung der Sensoroberfläche innerhalb weniger Minuten verfügbar.

In Ausführungsformen erfolgt die Detektion in Schritt f) durch mindestens zwei Streulichtmessungen, die beispielweise in einem zeitlichen Abstand von 30 Sekunden bis 1 Minute aufgenommen werden.

In alternativen Ausführungsformen erfolgt die Bestimmung der Bewegung des deformierbaren Partikels in Schritt f) durch mindestens zwei Messungen der Oberflächenplasmonenresonanz (SPR), *Total Internal Reflection* oder Fluoreszenz, die beispielweise in einem zeitlichen Abstand von 30 Sekunden bis 1 Minute aufgenommen werden.

In Ausführungsformen erfolgt weiterhin ein Vergleich mit mindestens einer Referenz und/oder eine Kalibrierung.

In Ausführungsformen erfolgt weiterhin eine parallele Messung einer Probe und einer Referenz.

Unter dem Begriff "Kalibrierung" wird eine Detektion verschiedener Standard-Lösungen des Analyten mit unterschiedlichen Konzentrationen mittels des erfindungsgemäßen Verfahrens und die Bestimmung einer Kalibriergerade verstanden. Vorteilhaft werden durch eine Kalibrierung instrumentelle Fehler und Matrixeffekte ausgeschlossen. In Ausführungsformen erfolgt die Kalibrierung mit einem internen Standard oder einem externen Standard.

Unter dem Begriff "Referenzwert" wird eine Probe ohne Analyten umfassend die gleiche Matrix verstanden. Unter dem Begriff "Matrix" werden die Bestandteile einer Analysenprobe verstanden, die nicht analysiert werden.

Ein weiterer Aspekt der Erfindung betrifft die Verwendung des Verfahrens zum Nachweis von Pestiziden, hormonell aktiven Verbindungen, Metallpartikeln, Metallionen, Viren, Bakterien, Biomarkern oder Antibiotika in Lebensmittel-, Futtermittel-, Waschmittel-, Spülmittel-, Körperpflegemittel-, Kosmetik-, Pharmaka-, Prozesswasser-, Boden-, Gewässer-, Trinkwasser- und/oder Abwasserproben und/oder Körperflüssigkeiten, wie Blutplasma-, Blutserum- oder Urinproben.

Unter dem Begriff "Prozesswasser" wird eine wässrige Lösung in technischen Produktionsprozessen verstanden. Zweckmäßig werden einzelne Parameter des Prozesswassers im Kreislauf untersucht.

In Ausführungsformen erfolgt die Verwendung des Verfahrens zum Nachweis von Antibiotika, bevorzugt Sulfonamiden, insbesondere Sulfamethoxazol oder Sulfanilamid, in Lebensmittel-, Futtermittel-, Waschmittel-, Spülmittel-, Körperpflegemittel-, Kosmetik-, Pharmaka-, Prozesswasser-, Boden-, Gewässer-, Trinkwasser- und/oder Abwasserproben und/oder Körperflüssigkeiten, wie Blutplasma-, Blutserum- oder Urinproben.

Ein weiterer Aspekt der Erfindung betrifft ein Kit umfassend:
i. mindestens eine Sensoroberfläche mit einem immobilisierten Analytbindungspartner,
   wobei der Analytbindungspartner fähig ist, mit einem Analyten zu interagieren, und
ii. mindestens ein deformierbares Partikel modifiziert mit einem Interaktionspartner,
dadurch gekennzeichnet, dass das deformierbare Partikel farbig ist oder eine farbige Markierung aufweist.

In Ausführungsformen umfasst das Kit mindestens zwei Sensoroberflächen, bevorzugt zwei bis 24 Sensoroberflächen, besonders bevorzugt zwei bis sechs Sensoroberflächen; umfassend jeweils einen immobilisierten Analytbindungspartner.

Zweckmäßig können die mindestens zwei Sensoroberflächen auf einem Sensor (Formkörper) als mindestens zwei verschiedene lokal begrenzte Teilbereiche oder zwei unterschiedliche Sensoren (Formkörper) ausgebildet sein. Zweckmäßig sind die mindestens zwei Sensoroberflächen parallel angeordnet, d. h. die Dimension der Sensoroberflächen, entlang derer die Bewegung der deformierbaren Partikel erfolgt, sind parallel zueinander angeordnet.

In Ausführungsformen ist der immobilisierte Analytbindungspartner auf der ersten Sensoroberfläche und der immobilisierte Analytbindungspartner auf der mindestens zweiten Sensoroberfläche gleich oder unterschiedlich.

In Ausführungsformen umfasst das Kit mindestens eine Sensoroberfläche zur Messung einer Probe, einer Negativkontrolle, einer Positivkontrolle und/oder einer Kalibrierreihe.

In Ausführungsformen umfasst das Kit mindestens zwei verschiedene deformierbare Partikel. In Ausführungsformen unterscheiden sich die mindestens zwei verschiedenen deformierbaren Partikel in Elastizitätsmodul, Partikeldurchmesser und/oder durch eine farbige Markierung oder unterschiedliche farbige Markierungen.

Für die Realisierung der Erfindung ist es auch zweckmäßig, die vorbeschriebenen erfindungsgemäßen Ausgestaltungen, Ausführungsformen und Merkmale der Ansprüche miteinander zu kombinieren.

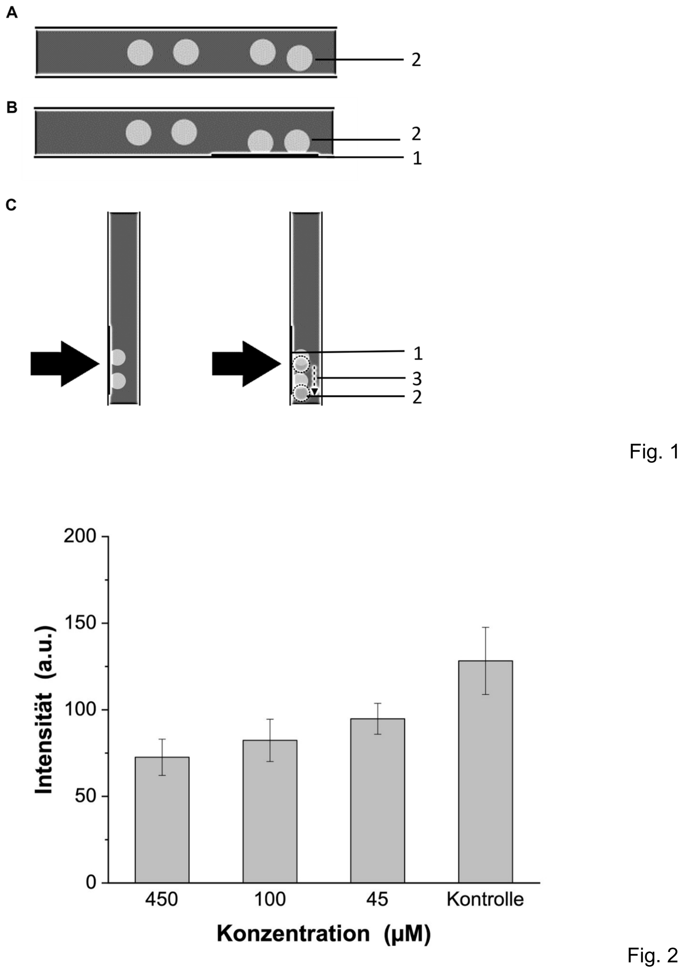

Nachfolgend soll die Erfindung anhand von Ausführungsbeispielen eingehender erläutert werden. Die Ausführungsbeispiele sollen dabei die Erfindung beschreiben, ohne diese zu beschränken. Anhand von Zeichnungen wird die Erfindung näher erläutert. Es zeigen die
**Fig. 1** eine schematische Darstellung des erfindungsgemäßen Verfahrens: **A)** Bereitstellung der deformierbaren Partikel (2), **B)** Bindung der deformierbaren Partikel auf der Sensoroberfläche (1): Bei einem komplexierenden Verfahren hohe Konzentration des Analyten, bei einem kompetitiven Verfahren geringe Konzentration des Analyten. **C)** Bewegung (3) der deformierbaren Partikel (2) entlang der Sensoroberfläche (1): Bei einem komplexierenden Verfahren geringe Konzentration des Analyten, bei einem kompetitiven Verfahren hohe Konzentration des Analyten.
**Fig. 2** die konzentrationsabhängige Modifizierung der deformierbaren Partikel mit Estradiol-17-(β-D-Glucuronid) (450, 100, 45 µM) für den Einsatz beim Nachweis von hormonell aktiven Analyten im Vergleich zu einer Positivkontrolle (Partikel mit Linker aber ohne Estradiol-17-(β-D-Glucuronid)-Modifizierung). Die Modifizierung der Partikel unter Nutzung eines 1-(2-(2-(2-aminoethoxy)ethoxy)ethyl)-1H-pyrrole-2,5-dion Linkers kann durch die Abnahme freier Amin-Gruppen am Linker nachgewiesen werden, welche durch eine Fluoreszenzmarkierung mittels Fluoresceinisothiocyanat erfolgt.

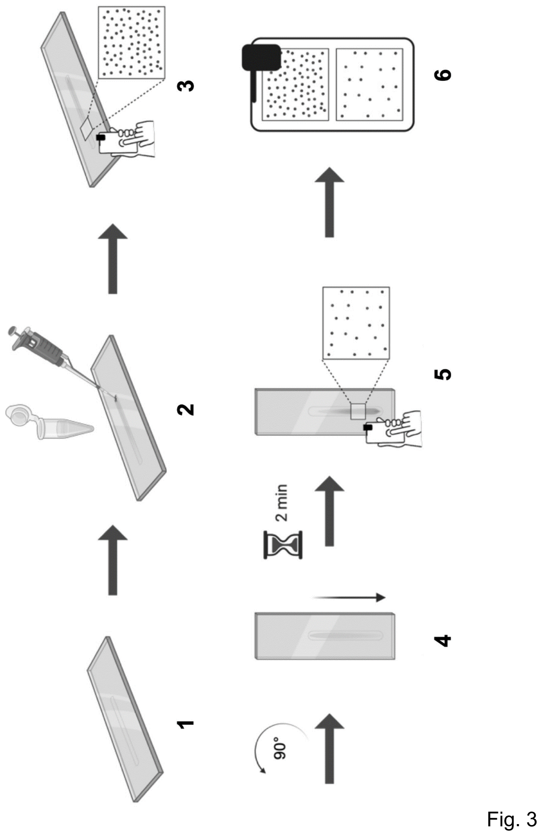

**Fig. 3** ein Ausführungsbeispiel der Durchführung des erfindungsgemäßen Verfahrens zur Detektion eines Analyten (hier 1 mM Sulfamethoxazol) bei kompetitiver Bindung mit dem Analytbindungspartner auf der Sensoroberfläche (hier Dihydropteroat-Synthase) mittels optischer Auslesung über ein Smartphone. 1) Sensoroberfläche mit abgedeckter Kanalstruktur: Die funktionalisierte Sensoroberfläche wird mittels doppelseitigen Klebebands und einem gereinigten Deckgläschen abgedeckt und zu einem Kanal präpariert. 2) In diesen Kanal als Sensoroberfläche in horizontaler Lage wird die Mischung aus Probe-Lösung und modifizierten Partikeln gegeben und eine Kontaktierung und Bindung der Partikel mit der Sensoroberfläche in horizontaler Lage für 1 bis 5 min realisiert und 3) die Partikel mittels Smartphone und Handymikroskop-Aufsatz optisch erfasst. 4) Anschließend erfolgt die Neigung der Sensoroberfläche in vertikale Position (90°) und 5) nach 2 min erneute Erfassung der adhärenten oder sich bewegenden Partikel auf der Sensoroberfläche mit dem Smartphone. 6) Anschließend erfolgt die Analyse des Anteils der bewegten Partikel zwischen den beiden Bildern: Aus der Differenz beider Bilder wird der Anteil sich bewegender Partikel berechnet, welcher ein Maß für die Analyt-Konzentration in der Probe ist.

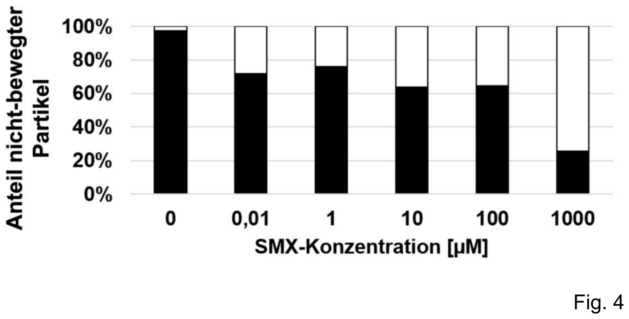

**Fig. 4** den Nachweis von Sulfamethoxazol in wässrigen Proben mittels des erfindungsgemäßen Verfahrens. In Abhängigkeit der Konzentration von Sulfamethoxazol im Konzentrationsbereich von 0,01 bis 1000 µM sinkt der Anteil der sich bewegenden Partikel bei einer vertikalen Neigung der Sensoroberfläche.

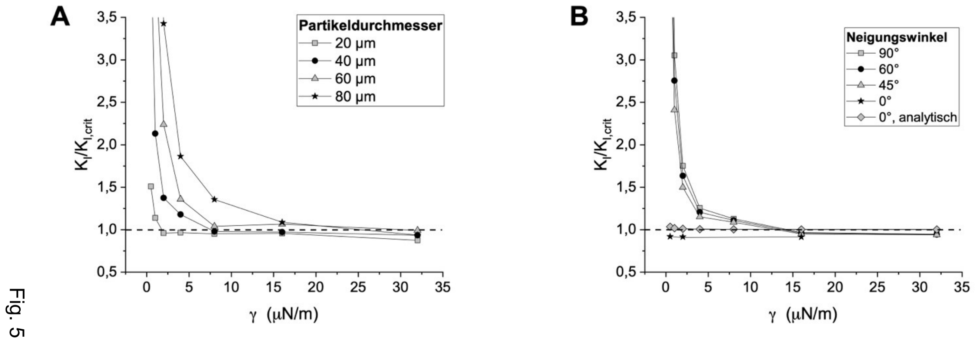

**Fig. 5** die Darstellung der Ergebnisse von Simulationen zur Abhängigkeit des Verhältnisses von Sedimentationskraft und Adhäsionskraft von den Verfahrensparametern Neigung der Sensoroberfläche, Bindungsstärke von modifiziertem Partikel und Sensoroberfläche sowie Durchmesser der Partikel auf das Ablösen und die Bewegung der Partikel. Das Verhältnis von Sedimentationskraft und Adhäsionskraft (hier über die Adhäsionsenergie γ zwischen Partikel und Sensoroberfläche ausgedrückt) ergibt sich aus dem dargestellten Parameter Kₗ/K_{l,crit}, welcher für Werte deutlich größer 1 ein Überschreiten der Scherrate über die kritische Scherrate angibt, welches zu einem Ablösen der Partikel führt. (A) Abhängigkeit für Kₗ/K_{l,crit} von der Adhäsionsenergie γ und dem Partikeldurchmesser, wobei deutliche Unterschiede für verschiedene Partikeldurchmesser zu sehen sind. (B) Abhängigkeit für Kₗ/K_{l,crit} von der Adhäsionsenergie γ und der Neigung der Sensoroberfläche, wobei keine deutlichen Unterschiede für verschiedene Neigungswinkel zu sehen sind. Für den 0° Winkel ist auch der Vergleich zu einer analytischen Lösung dargestellt, um die Simulationen zu bestätigen.

Das Prinzip der Nachweismethode ist in **Fig. 1** dargestellt. Die Probe, welche mit den modifizierten Partikeln vermischt wurde, wird mit der Sensoroberfläche in Kontakt gebracht (A), sodass die Analyten und die mit dem Interaktionspartner modifizierten Partikel (2) kompetitiv mit der mit dem Analytbindungspartner funktionalisierten Sensoroberfläche (1) wechselwirken können. Dabei befindet sich die Sensoroberfläche in horizontaler Lage, sodass die Sedimentationskraft auf die Mikropartikel deren schnelle Sedimentation und initiale Kontaktierung der Sensoroberfläche beschleunigt. In der Folge interagieren die Partikel in Abhängigkeit der Analyt-Konzentration in der Probe mit unterschiedlich starker Adhäsionskraft an der Sensoroberfläche (B). Nach 1 bis 5 min wird die Sensoroberfläche in vertikale Lage geneigt, sodass sich die Partikel im Falle einer geringen Adhäsionskraft durch die Sedimentationskraft bewegen (C, rechts). Im Falle einer hohen Adhäsionskraft bewegen sich die Partikel nicht (C, links). Der Anteil sich bewegender Partikel kann optisch mit verschiedenen Methoden ausgelesen und ausgewertet werden. Da die Adhäsionskraft abhängig von der Interaktion der modifizierten Partikel mit der Sensoroberfläche ist und diese Interaktion von der Analyt-Konzentration in der Probe abhängt, kann über den Anteil sich bewegender Partikel auf kalibrierte Grenzwerte der Analyt-Konzentration geschlossen werden.

### Herstellung deformierbarer Partikel aus Poly(ethylenglycol)hydrogelen mit und ohne farbliche Markierung durch Gold-Nanopartikel

Um monodisperse Hydrogelpartikel mit definierter Größe und Form zu erzeugen, wird ein mikrofluidisches Syntheseverfahren mittels Michael-Type Additionsreaktion von Maleimid- und Thiol-funktionalisierten Polyethylenglycol (PEG)-Precursor-Molekülen genutzt. Dafür wird ein 3-Spritzenpumpensystem mit mikrofluidischen Chips aus Polydimethylsiloxan (PDMS) mit entsprechend mikrostrukturierten Kanälen verwendet. Als Komponenten kommt ein 4-Arm-PEG-Thiol (MW: 2 kDa) und ein 4-Arm-PEG-Maleimid (MW 2 kDa) zum Einsatz, welche jeweils in 0,1 M Essigsäure (pH 4) mit einer Gewichtskonzentration von 12% (w/w) gelöst werden. Neben den PEG-Lösungen kommt 0,1 M Essigsäure als Synthese-Laufmittel sowie eine Ölphase, bestehend aus HFE-7500 Öl mit 2% (w/w) PicoSurf-Tensid zum Einsatz. Typische Flussraten des Setups liegen bei 800 µl/h für das Öl und 90 µl/h für das Laufmittel und die PEG-Lösungen, wodurch mit dem PDMS-Chip Partikel mit einem Durchmesser von 50 µm hergestellt werden, welche in einem Reaktionsgefäß von 1,5 ml gesammelt werden.

Zur Reinigung der Hydrogelpartikel werden 500 µl Reinstwasser zu der gesammelten Emulsion gegeben, wobei die Partikel in die wässrige Phase übergehen. Um die hergestellten Mikropartikel von der Öl-Tensid-Phase zu isolieren, werden 250 µl Perfluoroctanollösung (PFO, 20 % (v/v) in HFE (Ethyl 1,2,2,3,3,4,4,4-octafluoro-1-[1,2,2,2-tetrafluoro-1-(trifluoromethyl)ethyl]butylether)-7500) hinzugegeben. Die Emulsion Wasser/PFO-Mischung wird geschüttelt und 10 min bei 1840 g zentrifugiert. Das Öl wird entfernt und es werden erneut 250 µl Perfluoroctanollösung zugesetzt.

Die Partikelsuspension wird ein weiteres Mal zentrifugiert und die Perfluoroctanollösung entfernt. Die so erhaltenen Partikel können nun für die weitere Modifizierung genutzt werden.

Um farbige Mikropartikel herzustellen, werden 5 nm große Gold-Nanopartikel mit PEG-Methyl-Funktionalisierung (100 Da) während der Synthese eingeführt. Die Gold-Nanopartikel werden dafür vor der Synthese in das Laufmittel (0,1 M Essigsäure) und die PEG-Lösungen mit einer typischen Konzentration von 1 µM gegeben.

### Modifizierung der deformierbaren Partikel mit Sulfamethoxazol-Derivat

Die Modifizierung der farbigen oder nicht-farbigen Partikel erfolgt mit einem Sulfamethoxazol-Derivat (Tert-butyl(4-(N-(5-((2-mercaptoethyl)carbamoyl)isoxazol-3-yl)sulfamoyl)phenyl)-carbamat (SMXder)) über einen 1,11-Bis(maleimido)-3,6,9-trioxaundecan-Linker. Dafür werden 1 ml Partikelsuspension abzentrifugiert, 900 µl des Überstands verworfen und die verbleibende Suspension in 1 ml HEPES-Puffer resuspendiert. Nachfolgend erfolgt eine Reaktion für 1 h bei Raumtemperatur unter Rotation mit 3 mM 1,11-Bis(maleimido)-3,6,9-trioxaundecan in HEPES unter Zusatz von 5 % Dimethylsulfoxid (DMSO). Nach erneuter Zentrifugation, Entfernung des Überstands und Resuspendierung in 1 ml HEPES-Puffer folgt eine Reaktion für 1 h bei Raumtemperatur unter Rotation mit 6 mM SMXder-Lösung in Aceton/HEPES (1:3, v/v). Nach Zentrifugation und Resuspendierung in bidestilliertem Wasser erfolgt die Abspaltung der Boc-Schutzgruppe in 4 M HCl für 4 h bei Raumtemperatur unter Rotation. Anschließend erfolgt mehrfaches Zentrifugieren und Waschen in MOPS (3-(N-Morpholino)propansulfonsäure)-Puffer, in welchem die Lagerung für die Verwendung im Assay erfolgt.

### Konzentrationsabhängige Modifizierung der deformierbaren Partikel mit Estradiol 17-(β-D-Glucuronid) für den Einsatz beim Nachweis von hormonell aktiven Analyten

Die Modifizierung der Partikel erfolgte mit Estradiol 17-(β-D-Glucuronid) über einen 1-(2-(2-(2-Aminoethoxy)ethoxy)ethyl)-1H-pyrrol-2,5-dion-Linker. Dafür werden 1 ml Partikelsuspension zentrifugiert, 900 µl des Überstands verworfen und die verbleibende Suspension in 1 ml einer 3 mM 1-(2-(2-(2-Aminoethoxy)ethoxy)ethyl)-1H-pyrrol-2,5-dion-Lösung in 0,1 M HEPES-Puffer (pH 8,0) inkubiert. Die Reaktion der Linker an die Partikel erfolgt für 1 h bei Raumtemperatur unter Rotation. Nach Zentrifugation und Entfernung des Überstands erfolgt die Resuspendierung der Partikel in 1 ml 0,1 M MES (2-(N-Morpholino)ethansulfonsäure)-Puffer (pH 5,3). Nach erneuter Zentrifugation und Entfernung des Überstands erfolgt die Resuspendierung der Partikel in einer Estradiol-17-(β-D-Glucuronid)-Lösung in MES-Puffer mit DMSO (Dimethylsulfoxid, 9:1, v/v) und mit Zusatz von 40 mM 1-Ethyl-3-(3-dimethylaminopropyl)carbodiimid und N-Hydroxysulfosuccinimid. Die Reaktion mit Estradiol-17-(β-D-Glucuronid) erfolgt für 2 h bei Raumtemperatur unter Rotation. Estradiol-17-(β-D-Glucuronid) wird dabei in unterschiedlichen Konzentrationen (450 µM, 100 µM, 45 µM) zugesetzt, um eine unterschiedliche Modifizierung der Partikel zu erreichen. Anschließend erfolgen mehrfach Spülschritte mit Zentrifugation, Abnehmen des Überstands und Resuspendierung der Partikel in 0,1 M HEPES-Puffer (pH 8,0) mit DMSO (1:1, v/v). Abschließend erfolgt die Überführung der Partikel in 0,1 M HEPES-Puffer (pH 8,0). Der Nachweis der konzentrationsabhängigen Modifizierung der Partikel mit Estradiol-17-(β-D-Glucuronid) geschieht nach einer Maskierung freier Thiol-Gruppen mit N-Methylmaleimid und der Anfärbung mit Fluoresceinisothiocyanat (FITC) sowie anschließender Mikroskopie und Quantifizierung der mittleren Fluoreszenzintensität, da FITC an die freien Amingruppen des Linkers bindet, welche nicht durch die Kopplung von Estradiol 17-(β-D-Glucuronid) blockiert wurden. Die abnehmende Fluoreszenzintensität mit zunehmender Modifizierung der Partikel ist in **Fig. 2** gezeigt, auch mit Positivkontrolle (Partikel mit Linker aber ohne Estradiol-17-(β-D-Glucuronid)-Modifizierung). In Abhängigkeit der eingesetzten Estradiol-17-(β-D-Glucuronid)-Konzentration (450, 100, 45 µM) bei der Modifizierung der Partikel unter Nutzung eines 1-(2-(2-(2-Aminoethoxy)ethoxy)ethyl)-1H-pyrrol-2,5-dion-Linkers kann eine zunehmende Modifizierung durch die Abnahme freier Amin-Gruppen am Linker nachgewiesen werden.

### Funktionalisierung der Sensoroberfläche mit der Dihydropteroat-Synthase

Als Sensoroberflächen werden Deckgläser genutzt, welche zuvor mit destilliertem Wasser und Ethanol (96 %) für je 30 min mit Ultraschall bei Raumtemperatur gereinigt werden. Anschließend erfolgt ein weiterer Reinigungsschritt mit einer Lösung aus destilliertem Wasser, Wasserstoffperoxid und Ammoniak (25 %) im Verhältnis 5:1:1 (v/v/v) für 10 min bei 65 °C. Diese Glasoberflächen werden mit 20 mM 3-Aminopropyltriethoxysilan (APTES)-Lösung in Isopropanol für 10 min bei Raumtemperatur modifiziert, woran in einem weiteren Schritt eine Monolage von Poly(ethylen-*alt*-maleinsäureanhydrid) gebunden wird. Zur Kopplung des Analytbindungspartners, hier Dihydropteroat-Synthase (DHPS), wird die Polymerschicht bei 120 °C für 2 h aktiviert (Rezyklisierung von hydrolysierten Anhydrid-Gruppen) und mit 4 µM DHPS-Lösung in Phosphatpuffer für 1 h bei Raumtemperatur inkubiert. Anschließend erfolgt ein Spülen mit destilliertem Wasser und Trocknen mit Stickstoff.

### Nachweis des Sulfamethoxazol-Antibiotikums mittels kompetitiver Bindung und optischer Auslesung mit einem Smartphone

Für den Nachweis von Sulfamethoxazol (SMX) in einer wässrigen Probe werden in dem Verfahren entsprechend **Fig. 3** deformierbare PEG-Mikropartikel mit einer Färbung durch Gold-Nanopartikel und einer Modifizierung mit einem SMX-Derivat (SMXder) verwendet **(****Fig. 4****)**. Die Partikel werden in die Proben-Lösung in einem MOPS-Puffer mit Zusatz von 5 mM MgCl₂ und 0,6 mM Pyrophosphat gegeben, welche eine Konzentration von 0 bis 1 mM SMX besitzt. Die funktionalisierte Sensoroberfläche wurde mit doppelseitigem Klebeband und gegenüberliegendem gereinigtem Deckgläschen so präpariert, dass ein Abstand von 200 µm zwischen beiden Deckgläschen entsteht und ein Kanal von ungefähr 2 mm Breite. In diesen Kanal wurden 50 µl der Probe-Lösung pipettiert, welche sich durch die Kapillarwirkung auf der Sensoroberfläche verteilt. Nach 5 min erfolgte eine Bildaufnahme mithilfe eines Smartphones und angeschlossenem Handymikroskop-Aufsatzes mit einer Belichtungszeit von 2 s, wobei ein weißer diffuser Hintergrund genutzt wurde. Darauffolgend wird die Sensoroberfläche um 90° geneigt (vertikale Ausrichtung) und nach 2 min im gleichen Bereich wieder eine Bildaufnahme mit dem Smartphone durchgeführt. Mit computerbasierter Bildanalyse wird für die Partikel in beiden Bildern die Position bestimmt und über eine Differenzbildung herausgefunden, wie viele Partikel sich bewegen und wie viele Partikel sich nicht bewegen. In Abhängigkeit der Analyt-Konzentration (hier SMX) bewegen sich bei hoher Konzentration (hier 1 mM SMX) 75 % der Partikel. Allerdings können auch schon geringere Konzentrationen (hier 100 µM, 10 µM, 1 µM, 0,01 µM SMX) detektiert werden, wobei sich 25 % bis 40 % der Partikel bewegen im Vergleich zu einer Bewegung von nur 3 % der Partikel in einer Probe ohne Analyten (0 µM SMX). Damit kann eine Überschreitung des Grenzwerts von 1 mM SMX in der analysierten Probe gut bestimmt werden.

### Theoretische Analysen zur Abhängigkeit der Parameter Neigung, Bindungsstärke und Partikeldurchmesser auf das Verhältnis von Sedimentationskraft und Adhäsionskraft

In Simulationen zur Initiierung der Ablösung und Bewegung der Partikel bei der Neigung der Sensoroberfläche wird die Abhängigkeit des Verhältnisses von Sedimentationskraft und Adhäsionskraft von den Verfahrensparametern Neigung der Sensoroberfläche, Bindungsstärke von modifiziertem Partikel und Sensoroberfläche sowie Durchmesser der Partikel untersucht. Mittels Finite-Elemente-Methoden und physikalischen Modellen wird das Auftreten einer Bewegung von Partikeln auf geneigten Sensoroberflächen untersucht. Dabei wird unter Berechnung der Adhäsionsenergie γ und der Deformationsenergie unter dem Einfluss der Sedimentationskraft der Stressintensitätsfaktor Kₗ am Rand der Kontaktfläche eines Partikels berechnet. Entsprechend Theorien zur Elastizität und Rissbildung von Materialien kann ein kritischer Stressintensitätsfaktor K_{l,crit} in Abhängigkeit der Adhäsionsenergie berechnet werden. Ist Kₗ am Rand des Partikels größer als K_{l,crit}, kommt es zum Ablösen und zur Bewegung der Partikel. Somit können Werte von Kₗ/K_{l,crit} deutlich größer 1 als Kriterium zur Bewegung der Partikel bei Neigung der Sensoroberfläche genommen werden. Mit diesen Simulationen wird beobachtet **(****Fig. 5A****),** dass Kₗ/K_{l,crit} bei einer Variation der Adhäsionsenergie γ deutlich vom Durchmesser der Partikel abhängt, es also bei anderen Adhäsionsenergien zum Ablösen kommt.

Daraus wird abgeleitet, dass mit dem Durchmesser der Partikel die Empfindlichkeit und der Detektionsbereich des Verfahrens zum Nachweis von Analyten eingestellt werden kann. Die Abhängigkeit von Kₗ/K_{l,crit} von der Adhäsionsenergie γ bei unterschiedlichen Neigungen der Sensoroberfläche ändert sich jedoch kaum **(****Fig. 5B****),** sodass kleinere Schwankungen im Neigungswinkel (90° +/- 30°) zu keinen Änderungen bei dem Ablösen und der Bewegung der Partikel führen werden, wodurch eine Robustheit des Verfahrens hinsichtlich Neigungsschwankungen ergeben. Die Simulationen lassen sich mit einem Vergleich für einen Neigungswinkel 0° mit analytischen Lösungen des physikalischen Problems bestätigen.

### Zitierte Nichtpatentliteratur

Bittner M, Jarque S, Hilscherova K (2015) Polymer-immobilized ready-to-use recombinant yeast assays for the detection of endocrine disruptive compounds. Chemosphere, 132, 56-62.
Letarov AV und Kulikov EE (2017) Adsorption of Bacteriophages on Bacterial Cells. Biochemistry (Moscow) 82 (13), 1632-1658.
Rettke D, Danneberg C, Neuendorf TA, Kühn S, Friedrichs J, Hauck N, Werner C, Thiele J, Pompe T (2022) Microfluidics-assisted synthesis and functionalization of monodisperse colloidal hydrogel particles for optomechanical biosensors. J. Mater. Chem. B. 10, 1663-1674.
Rettke D, Döring J, Martin S, Venus T, Estrela-Lopis I, Schmidt S, Ostermann K, Pompe T (2020) Picomolar glyphosate sensitivity of an optical particle-based sensor utilizing biomimetic interaction principles. Biosens Bioelectron. 165,112262.
Rettke D, Seufert F, Döring J, Ostermann K, Wilms D, Schmidt S, Pompe T (2021) Biomimetic estrogen sensor based on soft colloidal probes. Biosensors and Bioelectronics 192, 113506.
Bertozzi Silva J, Storms Z, Sauvageau D (2016) Host receptors for bacteriophage adsorption. FEMS Microbiology Letters 363, fnw002.
Stone E, Campbell K, Grant I, McAuliffe O (2019) Understanding and Exploiting Phage-Host Interactions. Viruses 11, 567.

### Bezugszeichen

- 1: Sensoroberfläche
- 2: deformierbarer Partikel
- 3: Bewegung

## Patentansprüche

1. Verfahren zum Nachweis eines Analyten umfassend die Schritte:
a) Bereitstellen mindestens einer Sensoroberfläche (1) mit einem immobilisierten Analytbindungspartner,
wobei der Analytbindungspartner fähig ist, mit einem Analyten zu interagieren,
b) Bereitstellen einer Probe enthaltend den Analyten als Lösung oder Dispersion,
c) Kontaktieren der Sensoroberfläche (1) mit der Probe,
wobei der Analytbindungspartner mit dem Analyten interagiert,
d) Kontaktieren der Sensoroberfläche (1) mit einem deformierbaren Partikel (2), wobei das deformierbare Partikel (2) mit einem Interaktionspartner modifiziert ist, sodass in Abhängigkeit der Konzentration des Analyten eine Interaktion zwischen Partikel (2) und Sensoroberfläche (1) erfolgt,
e) Neigung der Sensoroberfläche (1) in einem Winkel im Bereich von 30° bis 150°, ausgehend von der horizontalen Achse der Sensoroberfläche (1), und
f) Detektion des Analyten durch optische Bestimmung der Bewegung (3) des deformierbaren Partikels (2) entlang der Sensoroberfläche (1).

2. Verfahren nach Anspruch 1, **dadurch gekennzeichnet, dass** die Sensoroberfläche (1) in Schritt e) in einem Winkel von etwa 90° ausgehend von der horizontalen Achse der Sensoroberfläche geneigt ist.

3. Verfahren nach Anspruch 1 oder 2, **dadurch gekennzeichnet, dass** der Analyt ein Pestizid, eine hormonell aktive Verbindung, Metallpartikel, Metallion, Virus, Bakterium, Biomarker oder Antibiotika ist.

4. Verfahren nach einem der Ansprüche 1 bis 3, **dadurch gekennzeichnet, dass** der Analytbindungspartner und der Interaktionspartner jeweils unabhängig voneinander aus einem Metall- oder Metallionen-bindenden Peptid oder Protein, einem Chelatbildner, einem Enzym, einem Membranprotein, einem Lipopolysaccharid oder einer Nukleinsäure ausgewählt ist.

5. Verfahren nach einem der Ansprüche 1 bis 4, **dadurch gekennzeichnet, dass** der Analytbindungspartner ausgewählt ist aus einer 5-Enolpyruvylshikimat-3-phosphat-Synthase-Domäne, einer Sulfotransferase oder einer Dihydropteroat-Synthase.

6. Verfahren nach einem der Ansprüche 1 bis 3, **dadurch gekennzeichnet, dass** der Interaktionspartner ausgewählt ist aus Glyphosat, Phosphoenolpyruvat, Phosametin oder einer Verbindung nach Formel (I) oder (II) 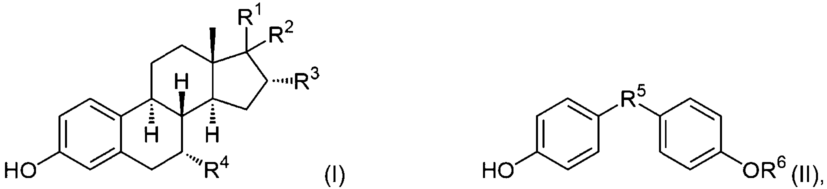
wobei R¹, R², R³, R⁴, R⁶ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen,
wobei R⁵ ausgewählt ist aus unsubstituierten oder substituierten C1- bis C15-Alkyl-, Alkenyl- oder Arylgruppen oder Kohlenhydratgruppen,
wobei der Interaktionspartner eine funktionelle Gruppe in Position R¹, R² oder R⁶ aufweist.

7. Verfahren nach einem der Ansprüche 1 bis 6, **dadurch gekennzeichnet, dass** das deformierbare Partikel (2) ein Hydrogelpartikel ist und/oder ein Elastizitätsmodul im Bereich von 1 kPa bis 50 kPa und/oder einen Durchmesser im Bereich von 10 µm bis 100 µm aufweist.

8. Verfahren nach einem der Ansprüche 1 bis 7, **dadurch gekennzeichnet, dass** das deformierbare Partikel (2) farbig ist oder eine farbige Markierung aufweist.

9. Verfahren nach einem der Ansprüche 1 bis 8, **dadurch gekennzeichnet, dass** der Interaktionspartner an das deformierbare Partikel (2) über einen Linker gebunden ist, wobei der Linker eine Konturlänge im Bereich von 5 Å bis 200 Å und/oder einen Polymerisationsgrad im Bereich von 1 bis 70 aufweist.

10. Verfahren nach einem der Ansprüche 1 bis 9, **dadurch gekennzeichnet, dass** die Detektion in Schritt f) visuell, mittels Durchlichtmikroskopie oder Streulichtmessung, erfolgt.

11. Verwendung eines Verfahrens nach einem der Ansprüche 1 bis 10 zum Nachweis von Pestiziden, hormonell aktiven Verbindungen, Metallpartikeln, Metallionen, Viren, Bakterien, Biomarkern oder Antibiotika in Lebensmittel-, Futtermittel-, Waschmittel-, Spülmittel-, Körperpflegemittel-, Kosmetik-, Pharmaka-, Prozesswasser-, Boden-, Gewässer-, Trinkwasser- und/oder Abwasserproben und/oder Körperflüssigkeiten.

12. Kit umfassend:
i. mindestens eine Sensoroberfläche (1) mit einem immobilisierten Analytbindungspartner,
wobei der Analytbindungspartner fähig ist, mit einem Analyten zu interagieren, und
ii. mindestens ein deformierbares Partikel (2) modifiziert mit einem Interaktionspartner,
**dadurch gekennzeichnet, dass** das deformierbare Partikel (2) farbig ist oder eine farbige Markierung aufweist.

13. Kit nach Anspruch 12, **dadurch gekennzeichnet, dass** das deformierbare Partikel (2) ein Hydrogelpartikel ist und/oder ein Elastizitätsmodul im Bereich von 1 kPa bis 50 kPa und/oder einen Durchmesser im Bereich von 10 µm bis 100 µm aufweist.

14. Kit nach Anspruch 12 oder 13, **dadurch gekennzeichnet, dass** der Analytbindungspartner und der Interaktionspartner jeweils unabhängig voneinander aus einem Metall- oder Metallionen-bindenden Peptid oder Protein, einem Chelatbildner, einem Enzym, einem Membranprotein, einem Lipopolysaccharid oder einer Nukleinsäure ausgewählt ist.

15. Kit nach Anspruch 12 oder 13, **dadurch gekennzeichnet, dass** der Analytbindungspartner aus einem Metall- oder Metallionen-bindenden Peptid oder Protein, einem Chelatbildner, einem Enzym, einem Membranprotein, einem Lipopolysaccharid oder einer Nukleinsäure ausgewählt ist und der Interaktionspartner ausgewählt ist aus Glyphosat, Phosphoenolpyruvat, Phosametin oder einer Verbindung nach Formel (I) oder (II) 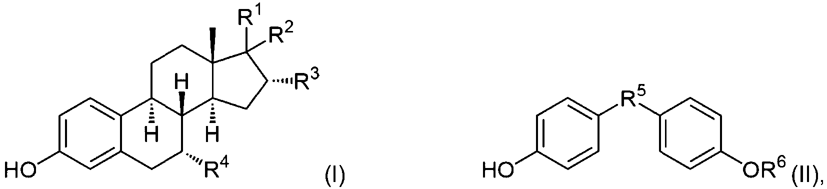
wobei R¹, R², R³, R⁴, R⁶ jeweils unabhängig voneinander ausgewählt ist aus H, funktionellen Gruppen, unsubstituierten oder substituierten C1- bis C15-Alkyl-, Alkenyl- oder Arylgruppen oder Kohlenhydratgruppen,
wobei R⁵ ausgewählt ist aus unsubstituierten oder substituierten C1- bis C15-Alkyl-, Alkenyl- oder Arylgruppen oder Kohlenhydratgruppen,
wobei der Interaktionspartner eine funktionelle Gruppe in Position R¹, R² oder R⁶ aufweist.
